# EUROPÄISCHE PATENTSCHRIFT

(11) **EP 4 095 865 B1**
(45) Veröffentlichungstag und Bekanntmachung des Hinweises auf die Patenterteilung: **29.10.2025**
(21) Anmeldenummer: 21175587.1
(22) Anmeldetag: 25.05.2021
(51) Int. Cl.: G16H 30/40, G16H 50/20

(54) **VERFAHREN ZUM ZUORDNEN EINES MEDIZINISCHEN BILDDATENSATZES ZU EINEM MEDIZINISCHEN VERGLEICHSBILDDATENSATZ IN ABHÄNGIGKEIT VON BIOMETRISCHEN DATEN**
METHOD FOR ASSIGNING A MEDICAL IMAGE DATA SET TO A MEDICAL COMPARISON IMAGE DATA SET AS A FUNCTION OF BIOMETRIC DATA
PROCÉDÉ D'AFFECTATION D'UN ENSEMBLE DE DONNÉES D'IMAGES MÉDICALES À UN ENSEMBLE DE DONNÉES D'IMAGES MÉDICALES COMPARATIVES EN FONCTION DES DONNÉES BIOMÉTRIQUES

(43) Veröffentlichungstag der Anmeldung: 30.11.2022
(73) Patentinhaber: Siemens Healthineers AG, 91301 Forchheim (DE)
(72) Erfinder: Sperl, Jonathan, 96047 Bamberg (DE); Zenge, Michael, 90419 Nürnberg (DE); Kaftan, Jens, 96047 Bamberg (DE)
(74) Vertreter: Siemens Healthineers Patent Attorneys

(56) Entgegenhaltungen:
- US-A1- 2010 307 516

## Beschreibung

In der medizinischen Bildgebung wird häufig eine Mehrzahl von medizinischen Bilddatensätzen von einem Patienten erfasst. Die medizinischen Bilddatensätze können dabei zu verschiedenen Zeitpunkten erfasst worden sein. Typischerweise umfasst dabei jeder Bilddatensatz eine Abbildung bzw. ein medizinisches Bild wenigstens eines Teils des Patienten. Insbesondere kann dabei wenigstens ein Teil der Mehrzahl von medizinischen Bilderdatensätzen ein medizinisches Bild einer Pathologie, beispielsweise eines Tumors, des Patienten umfassen. Der Teil der Mehrzahl von medizinischen Bilddatensätzen kann dabei eine zeitliche Veränderung der Pathologie abbilden. Mit anderen Worten kann anhand des Teils der Mehrzahl von medizinischen Bilddatensätzen eine longitudinale bzw. zeitliche Analyse einer Veränderung der Pathologie durchgeführt werden.

Typischerweise werden in einer medizinischen Einrichtung beispielsweise einer Klinik und/oder einer Arztpraxis eine Mehrzahl von Bilddatensätzen von einer Mehrzahl von Patienten erfasst, gespeichert und/oder analysiert.

Um eine einfache Analyse der medizinischen Bilddatensätze zu ermöglichen, ist es notwendig, die medizinischen Bilddatensätze eines Patienten einander zuordnen zu können. Insbesondere ist es vorteilhaft, dass ein Nutzer auswählen kann für welchen Patienten die Analyse durchgeführt werden soll und automatisch die medizinischen Bilddatensätze, die dem Patienten zugeordnet sind, bei der Analyse berücksichtigt werden. Wird ein neuer medizinsicher Bilddatensatz des Patienten erfasst, soll dieser der Mehrzahl von medizinischen Bilddatensätzen, die bereits von dem Patienten erfasst wurden und bereits einander zugeordnet sind, zugeordnet werden.

Gleichzeitig soll sichergestellt werden, dass ein medizinischer Bilddatensatz von einem anderen Patienten nicht fälschlicherweise in der Analyse berücksichtigt werden.

Außerdem wäre es vorteilhaft, wenn der Nutzer informiert würde, wenn der Unterschied zwischen den einzelnen, einander zugeordneten medizinischen Bilddatensätzen des einen Patienten zu groß ist, um eine sinnvolle longitudinale Analyse durchzuführen. Ein zu großer Unterschied bzw. eine große Veränderung zwischen den medizinischen Bilddatensätzen kann darauf hindeuten, dass der Patient sich stark verändert hat, beispielsweise durch eine Gewichtsveränderung und/oder durch eine anormale Veränderung der Pathologie. Alternativ kann ein zu großer Unterschied bzw. Veränderung darauf hindeuten, dass wenigstens einer der einander zugeordneten medizinischen Bilddatensätze von einem anderen Patienten ist bzw. ein medizinisches Bild eines anderen Patienten umfasst.

Die Mehrzahl der medizinischen Bilddatensätze eines Patienten werden typischerweise in einer Datenbank hinterlegt. Typischerweise umfasst die Datenbank medizinische Bilddaten von einer Mehrzahl an Patienten. Die Analyse der medizinischen Bilddatensätze erfolgt häufig in einem Cloudsystem. Dafür werden die zu analysierenden Bilddatensätze in das Cloudsystem hochgeladen.

Es ist bekannt, dass ein medizinischer Bilddatensatz Patientendaten umfassen kann. Über die Patientendaten kann der Patient, dessen medizinisches Bild bzw. Abbildung der medizinische Bilddatensatz umfasst, identifiziert werden. Typischerweise werden diese Patientendaten von einem DICOM (Akronym für Digital Imaging and Communications in Imaging) Header umfasst. Die Patientendaten umfassen beispielsweise einen Namen, ein Geburtsdatum und/oder eine Patienten-Identifikationsnummer (beispielsweise eine Krankenkassenidentifikationsnummer) des Patienten. Es ist bekannt, die medizinischen Bilddatensätze eines Patienten basierend auf diesen Patientendaten manuell oder automatisiert einander zuzuordnen.

In einer Notfallsituation werden häufig die Patientendaten nicht korrekt erfasst, was eine Zuordnung des in der Notfallsituation erfassten medizinischen Bilddatensatzes zu einem bereits im Vorfeld erfassten medizinischen Bilddatendatensatz desselben Patienten häufig unmöglich oder aufwendig macht.

Um den Datenschutz zu gewährleisten, wird ein medizinischer Bilddatensatz spätestens vor dem Hochladen in das Cloudsystem pseudonymisiert oder anonymisiert. In Ausführungen sind die medizinischen Bilddatensätze bereits in der Datenbank pseudonymisiert oder anonymisiert. Beim Pseudonymisieren bzw. Anonymisieren eines medizinischen Bilddatensatzes werden Patientendaten, die Rückschlüsse auf die Identität des Patienten zulassen, durch ein Pseudonym ersetzt bzw. aus dem medizinischen Bilddatensatz entfernt.

Aus diesem Grund ist es häufig nicht möglich die pseudonymisierten bzw. anonymisierten medizinischen Bilddatensätze eines Patienten basierend auf den Patientendaten einander zuzuordnen.

Alternativ können die Patientendaten vor dem Hochladen in das Cloudsystem oder bereits in der Datenbank verschlüsselt sein. Dabei werden die Patientendaten medizinischer Bilddatensätze, die mit unterschiedlichen Bildgebungssystemen erfasst wurden, häufig mittels unterschiedlicher Algorithmen verschlüsselt. Somit ist es nicht möglich die auf verschiedene Weise verschlüsselten Patientendaten einander zuzuordnen.

Es ist bekannt, dass der Nutzer die medizinischen Bilddatensätze des Patienten, die analysiert werden sollen, in diesen Fällen manuell auswählt. Mit anderen Worten kann der Nutzer die medizinischen Bilddatensätze eines Patienten manuell einander zuordnen. Dieses Vorgehen ist allerdings sehr fehleranfällig.

Die US 2010/0307516 A1 beschreibt ein Verfahren zum Bestimmen der Körperregion, in der ein anatomischer Körperteil eines Patienten angeordnet ist.

Es ist daher die Aufgabe der vorliegenden Erfindung, eine Methode bereitzustellen, die eine Zuordnung medizinischer Bilddatensätze eines Patienten unabhängig von den Patientendaten und zuverlässig ermöglicht.

Die Aufgabe wird gelöst durch ein Verfahren zum Zuordnen eines medizinischen Bilddatensatzes zu einem medizinischen Vergleichsbilddatensatz in Abhängigkeit von biometrischen Daten, durch eine Vorrichtung zum Zuordnen eines medizinischen Bilddatensatzes zu einem medizinischen Vergleichsbilddatensatz in Abhängigkeit von biometrischen Daten, durch ein Computerprogrammprodukt und durch ein computerlesbares Speichermedium gemäß den unabhängigen Ansprüchen. Vorteilhafte Weiterbildungen sind in den abhängigen Ansprüchen und in der folgenden Beschreibung aufgeführt.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung betrifft ein computerimplementiertes Verfahren zum Zuordnen eines medizinischen Bilddatensatzes zu einem medizinischen Vergleichsbilddatensatz in Abhängigkeit von biometrischen Daten. Das Verfahren umfasst einen Verfahrensschritt eines Empfangens des medizinischen Bilddatensatzes.

Das Verfahren umfasst weiterhin einen Verfahrensschritt eines Empfangens eines medizinischen Vergleichsbilddatensatzes. Das Verfahren umfasst weiterhin einen Verfahrensschritt eines Extrahierens von biometrischen Daten basierend auf dem medizinischen Bilddatensatz. Das Verfahren umfasst weiterhin einen Verfahrensschritt eines Extrahierens von biometrischen Vergleichsdaten basierend auf dem medizinischen Vergleichsbilddatensatz. Das Verfahren umfasst weiterhin einen Verfahrensschritt eines Bestimmens eines Abweichungsmaßes zwischen den biometrischen Vergleichsdaten und den biometrischen Daten. Das Verfahren umfasst weiterhin einen Verfahrensschritt eines Zuordnens des medizinischen Bilddatensatzes zu dem medizinischen Vergleichsbilddatensatz, wenn das Abweichungsmaß einen Schwellenwert unterschreitet.

Der medizinische Bilddatensatz umfasst eine Abbildung eines Patienten. Insbesondere umfasst der medizinische Bilddatensatz eine Abbildung eines Teils des Patienten. Der Teil des Patienten kann insbesondere ein Körperteil beispielsweise ein Kopf, ein Thorax, ein Abdomen, ein Arm und/oder ein Bein etc. des Patienten sein. Die Abbildung ist dabei ein medizinisches Bild des Patienten bzw. des Teils des Patienten. Im Folgenden wird die Bezeichnung "der medizinische Bilddatensatz bildet den Patienten ab" synonym zu "das von dem medizinischen Bilddatensatz umfasste medizinische Bild bildet einen Teil des Patienten bzw. den Patienten ab" verwendet. Der medizinische Bilddatensatz wird dabei mit einem medizinischen Bildgebungssystem erfasst. Das medizinische Bildgebungssystem kann insbesondere ein Röntgen-System, ein Mammographie-System, ein Computer-Tomographie-System, ein Magnet-Resonanz-Tomographie-System, ein C-Bogen-System, ein Ultraschall-System, ein Positronen-Emissions-Tomographie-System oder ein Einzel-Photonen-Emissions-Computer-Tomographie-System etc. sein. Die Abbildung bzw. das medizinische Bild kann somit ein pixeliertes oder voxeliertes Bild sein. Mit anderen Worten umfasst das medizinische Bild eine zweidimensionale Pixelmatrix oder eine dreidimensionale Voxelmatrix. Dabei ist jedem Pixel bzw. Voxel ein Pixelwert bzw. Voxelwert zugeordnet. Mit anderen Worten kann das medizinische Bild ein dreidimensionales oder ein zweidimensionales medizinisches Bild sein. Der medizinische Bilddatensatz kann insbesondere anonymisiert oder pseudonymisiert oder verschlüsselt sein. Mit anderen Worten kann der medizinische Bilddatensatz derart ausgebildet sein, dass er keine Patientendaten umfasst, die eine Identifizierung des in dem von dem medizinischen Bilddatensatz umfassten medizinischen Bild dargestellten Patienten erlaubt. Die Patientendaten können beispielsweise einen Namen, ein Geschlecht, ein Geburtsdatum, eine Adresse, eine patientenspezifische Identifikationsnummer etc. umfassen. Bei eine Pseudonymisierung werden die Patientendaten, die Rückschlüsse auf den Patienten erlauben, durch einen generischen Wert ersetzt. Bei der Anonymisierung werden die Patientendaten, die Rückschlüsse auf den Patienten erlauben, entfernt bzw. gelöscht. Bei der Verschlüsselung werden die Patientendaten, die Rückschlüsse auf den Patienten erlauben, derart verschlüsselt, dass nur mit Hilfe wenigstens eines Schlüssels die Patientendaten in Klartextdaten überführbar sind. Die Verschlüsselung kann von dem Bildgebungssystem abhängen, mit dem der medizinische Bilddatensatz erfasst wurde.

Der medizinische Vergleichsbilddatensatz ist analog zu dem medizinischen Bilddatensatz ausgebildet. Der medizinische Vergleichsbilddatensatz kann dabei ein medizinisches Bild bzw. eine Abbildung desselben Patienten wie der medizinische Bilddatensatz umfassen. Alternativ kann der medizinische Vergleichsbilddatensatz ein medizinisches Bild eines anderen Patienten als der medizinische Bilddatensatz umfassen. Der medizinische Vergleichsbilddatensatz kann in einem medizinischen Bild desselben Teils eines Patienten abbilden wie der medizinische Bilddatensatz. Alternativ kann der medizinische Vergleichsbilddatensatz einen anderen Teil eines Patienten abbilden als der medizinische Bilddatensatz. Der medizinische Vergleichsbilddatensatz kann mit demselben oder einem anderen Bildgebungssystem erfasst worden sein wie der medizinische Bilddatensatz. Im Folgenden wird die Bezeichnung "der medizinische Vergleichsbilddatensatz bildet den Patienten ab" synonym zu "das von dem medizinischen Vergleichsbilddatensatz umfasste medizinische Bild bzw. Abbildung bildet einen Teil des Patienten bzw. den Patienten ab" verwendet. Der medizinische Vergleichsbilddatensatz kann insbesondere pseudonymisiert oder anonymisiert oder verschlüsselt sein.

In den Verfahrensschritten des Empfangens des medizinischen Bilddatensatzes und des Empfangens wenigstens eines medizinischen Vergleichsbilddatensatzes werden der medizinische Bilddatensatz und der medizinische Vergleichsbilddatensatz mittels einer Schnittstelle empfangen. Der medizinische Bilddatensatz und/oder der medizinische Vergleichsbilddatensatz können dabei von dem medizinischen Bildgebungssystem bereitgestellt werden, mit dem sie erfasst wurden. Mit anderen Worten können der medizinische Bilddatensatz und/oder der medizinische Vergleichsbilddatensatz mittels der Schnittstelle von dem entsprechenden medizinischen Bildgebungssystem empfangen werden. Alternativ können der medizinische Bilddatensatz und/oder der medizinische Vergleichsbilddatensatz von einer Datenbank bereitgestellt werden. Mit anderen Worten können der medizinische Bilddatensatz und/oder der medizinische Vergleichsbilddatensatz mittels der Schnittstelle von der Datenbank empfangen werden. Insbesondere können der medizinische Vergleichsbilddatensatz und/oder der medizinische Bilddatensatz in der Datenbank hinterlegt bzw. gespeichert sein. Die Datenbank kann insbesondere auf einem lokalen Server oder in einem Cloudsystem hinterlegt bzw. gespeichert sein. Die Datenbank kann dabei insbesondere ein Bildarchivierungs- und Kommunikationssystem (engl.: Picture Archiving and Communication System, Akronym: PACS), ein Krankenhausinformationssystem (Akronym: KIS) und/oder ein Radiologieinformationssystem (Akronym: RIS) etc. sein.

Im Folgenden verweist die Bezeichnung "Bilddatensätze" auf den medizinischen Bilddatensatz und dem medizinischen Vergleichsbilddatensatz.

In dem Verfahrensschritt des Extrahierens von biometrischen Daten werden die biometrischen Daten basierend auf dem medizinischen Bilddatensatz extrahiert. Mit anderen Worten werden die biometrischen Daten in Abhängigkeit des medizinischen Bilddatensatzes extrahiert. Dabei kann der medizinische Bilddatensatz die biometrischen Daten umfassen. In dem Schritt des Extrahierens der biometrischen Daten werden diese dann aus dem medizinischen Bilddatensatz ausgelesen. Alternativ können die biometrischen Daten basierend auf dem medizinischen Bilddatensatz bestimmt werden. Mit anderen Worten kann das Extrahieren der biometrischen Daten ein Bestimmen der biometrischen Daten umfassen. Insbesondere können die biometrischen Daten in Abhängigkeit der von dem medizinischen Bilddatensatz umfassten Abbildung bzw. medizinischen Bild bestimmt werden. Insbesondere können die biometrischen Daten basierend auf bzw. aus den in den in dem medizinischen Bilddatensatz bzw. in dem medizinischen Vergleichsbilddatensatz enthaltenen Bilddaten bzw. medizinischem Bild bzw. Abbildung ermittelt, extrahiert und/oder berechnet werden. Insbesondere können hierfür computer-implementierte Mittel bzw. Verfahren zur, insbesondere automatischen, Bilddatenanalyse verwendet werden, die gemäß Ausführungsbeispielen als Computerprogrammprodukte implementiert sein können.

Die biometrischen Daten können insbesondere eine anatomische Ausprägung des Patienten, dessen medizinisches Bild von dem medizinischen Bilddatensatz umfasst ist, beschreiben. Wie ein Fingerabdruck eine individuelle, einmalige Ausprägung der Hautstruktur an einem Finger beschreibt, beschreiben die biometrischen Daten eine individuelle, einmalige Ausprägung der Anatomie des Patienten. Beispielsweise können die biometrischen Daten eine Form eines Organs und/oder eine Lage verschiedener Organe zueinander und/oder eine Ausprägung eines Skeletts bzw. eines Teils eines Skeletts und/oder einer Form von Hirnwindungen etc. beschreiben bzw. umfassen. In alternativen Ausführungen können die biometrischen Daten wenigstens eine Besonderheit des Patienten angeben. Beispielsweise können die biometrischen Daten angeben, wenn der Patient nur eine Niere umfasst und/oder ein Knochenbruch und/oder eine aus dem medizinischen Bild ableitbare Größe des Patienten etc.

In dem Verfahrensschritt des Extrahierens der biometrischen Vergleichsdaten werden die biometrischen Vergleichsdaten basierend auf dem medizinischen Vergleichsbilddatensatz extrahiert. Mit anderen Worten werden die biometrischen Vergleichsdaten in Abhängigkeit des medizinischen Vergleichsbilddatensatzes extrahiert. Das Extrahieren kann dabei wie bezüglich der biometrischen Daten beschrieben ausgebildet sein. Insbesondere kann das Extrahieren ein Auslesen oder ein Bestimmen der biometrischen Vergleichsdaten umfassen.

Die biometrischen Vergleichsdaten sind dabei analog zu den biometrischen Daten ausgebildet. Die biometrischen Vergleichsdaten beschreiben dabei eine individuelle, einmalige Ausprägung der Anatomie des in dem von dem medizinischen Vergleichsbilddatensatzes umfassten medizinischen Bild abgebildeten Patienten.

In dem Verfahrensschritt des Bestimmens des Abweichungsmaßes wird das Abweichungsmaß zwischen den biometrischen Vergleichsdaten und den biometrischen Daten bestimmt. Das Abweichungsmaß beschreibt dabei, wie stark die biometrischen Daten von den biometrischen Vergleichsdaten abweichen. Das Abweichungsmaß kann beispielsweise in Prozent angeben, wie groß die Abweichung ist. Alternativ oder zusätzlich kann das Abweichungsmaß die Abweichung als reelle Zahl angeben, die eine Abweichung zwischen den biometrischen Daten und den biometrischen Vergleichsdaten beschreibt. Beispielsweise kann die reelle Zahl eine Abweichung in Millimeter oder Zentimeter angeben. Alternativ oder zusätzlich kann das Abweichungsmaß die Abweichung in kategorisierter Form angeben. Beispielsweise kann die Abweichung zwischen den biometrischen Daten und den biometrischen Vergleichsdaten in die Kategorien bzw. Klassen "große Abweichung", "mittel", "kaum Abweichungen" oder "3", "2", "1" unterteilt sein. Das Abweichungsmaß gibt dann die Kategorie an, in die die Abweichung der biometrischen Daten und der biometrischen Vergleichsdaten eingeteilt bzw. kategorisiert wurde. Die Kategorien können dabei hierarchisch angeordnet sein. Dabei können die Kategorien, die für eine kleine Abweichung stehen unter den Kategorien, die für eine große Abweichung stehen angeordnet sein.

In dem Verfahrensschritt des Zuordnens des medizinischen Bilddatensatzes zu dem medizinischen Vergleichsbilddatensatz, wird der medizinischen Bilddatensatz dem medizinischen Vergleichsbilddatensatz zugeordnet, wenn das Abweichungsmaß den Schwellenwert unterschreitet.

Der Schwellenwert gibt an, wie groß das Abweichungsmaß maximal sein darf, damit davon ausgegangen werden kann, dass das von dem medizinische Bilddatensatz umfasste medizinische Bild und das von dem medizinischen Vergleichsbilddatensatz umfasste medizinische Bild denselben Patienten abbilden. Insbesondere gibt der Schwellenwert an, wie groß das Abweichungsmaß maximal sein darf, damit eine weitere Analyse insbesondere ein Vergleich des medizinischen Bildes des medizinischen Bilddatensatzes und des medizinischen Bildes des medizinischen Vergleichsbilddatensatzes möglich ist. Der Schwellenwert kann abhängig von der Ausprägung des Abweichungsmaßes einen Prozentwert, eine reelle Zahl und/oder eine Kategorie angeben. Der Schwellenwert kann insbesondere empirisch bestimmt worden sein.

Wenn das Abweichungsmaß den Schwellenwert unterschreitet, wird der medizinische Bilddatensatz dem medizinischen Vergleichsbilddatensatz zugeordnet. Wenn das Abweichungsmaß den Schwellenwert unterschreitet, kann davon ausgegangen werden, dass der medizinische Bilddatensatz und der medizinische Vergleichsbilddatensatz denselben Patienten abbilden. Mit anderen Worten bilden die von dem medizinischen Bilddatensatz und dem medizinischen Vergleichsbilddatensatz umfassten medizinischen Bilder denselben Patienten ab, wenn das Abweichungsmaß den Schwellenwert unterschreitet.

Das Zuordnen bedeutet dabei, dass für eine nachfolgende Bearbeitung oder Analyse oder Anzeigen des medizinischen Bilddatensatzes und des medizinischen Vergleichsbilddatensatzes hinterlegt ist, dass beide Bilddatensätze denselben Patienten abbilden. Mit anderen Worten gibt die Zuordnung an, dass der medizinische Bilddatensatz und der medizinische Vergleichsbilddatensatz miteinander in Beziehung stehen. Beispielsweise kann dafür eine Verknüpfung des medizinischen Bilddatensatzes und des medizinischen Vergleichsbilddatensatzes in der Datenbank hinterlegt werden. Insbesondere kann das Zuordnen bewirken, dass beim Aufrufen eines Bilddatensatzes automatisch der zugeordnete Bilddatensatz ebenfalls aufgerufen wird. Mit anderen Worten wird der medizinische Bilddatensatz aufgerufen, wenn der medizinische Vergleichsbilddatensatz aufgerufen wird und der medizinische Bilddatensatz dem medizinischen Vergleichsbilddatensatz zugeordnet ist. Aufrufen kann hierbei bedeuten, dass der aufgerufene Bilddatensatz für eine Bearbeitung und/oder eine Analyse und/oder eine Anzeige verwendet werden soll.

Die Erfinder haben erkannt, dass von einem medizinischen Bilddatensatz biometrische Daten extrahiert werden können. Dabei basieren die biometrischen Daten auf einer von dem Bilddatensatz umfassten Abbildung bzw. medizinischen Bild. Die Erfinder haben erkannt, dass ein Patient anhand solcher biometrischen Daten identifizierbar ist. Die Erfinder haben somit erkannt, dass anhand der biometrischen Daten in dem medizinischen Bild des medizinischen Bilddatensatzes und der biometrischen Vergleichsdaten des medizinischen Vergleichsbilddatensatzes erkannt werden kann, ob beide medizinischen Bilder denselben Patienten abbilden. Die Erfinder haben erkannt, dass auf diese Weise automatisiert bestimmt werden kann, ob die Bilddatensätze denselben Patienten oder einen ähnlichen Patienten abbilden, mit dem ein Vergleich mit dem zu befundenden Patienten sinnvoll ist. Die Erfinder haben erkannt, dass dies unabhängig davon passieren kann, ob von den Bilddatensätzen umfasste Patientendaten anonymisiert oder pseudonymisiert oder verschlüsselt sind. Mit anderen Worten ist eine Zuordnung von Bilddatensätzen, die denselben Patienten abbilden ohne die Patientendaten möglich.

Nach einem Aspekt der Erfindung bildet der medizinische Vergleichsbilddatensatz wenigstens einen Körperteil eines ersten Patienten ab. Alternativ oder zusätzlich bildet der medizinischen Bilddatensatz wenigstens einen Körperteil des ersten Patienten oder eines zweiten Patienten ab.

Wie oben beschrieben bedeutet "der medizinische Vergleichsbilddatensatz bildet wenigstens einen Körperteil ab", dass der medizinische Vergleichsbilddatensatz ein medizinisches Bild umfasst, welches den wenigstens einen Körperteil abbildet. Analog bedeutet "der medizinische Bilddatensatz bildet wenigstens einen Körperteil ab", das der medizinische Bilddatensatz ein medizinisches Bild umfasst, das den wenigstens einen Körperteil abbildet.

Der wenigstens eine Körperteil kann wenigstens ein Teil des ersten bzw. zweiten Patienten sein. Insbesondere kann der wenigstens eine Körperteil der ganze erste bzw. zweite Patient sein. Alternativ kann der wenigstens eine Körperteil beispielsweise der Kopf, der Thorax, das Abdomen, ein Bein, ein Arm etc. des ersten bzw. zweiten Patienten sein. Insbesondere kann der medizinische Vergleichsbilddatensatz oder der medizinische Bilddatensatz mehr als einen Körperteil abbilden. Beispielsweise kann der medizinische Vergleichsbilddatensatz oder der medizinische Bilddatensatz den Thorax und das Abdomen des ersten bzw. zweiten Patienten abbilden.

Insbesondere können der medizinische Vergleichsbilddatensatz und der medizinische Bilddatensatz wenigstens ein Körperteil des gleichen, insbesondere des ersten Patienten, abbilden. Dabei kann der medizinische Vergleichsbilddatensatz und der medizinische Bilddatensatz den gleichen wenigstens einen Körperteil des ersten Patienten abbilden. Alternativ kann der medizinische Vergleichsbilddatensatz wenigstens einen anderen Körperteil des ersten Patienten abbilden als der medizinische Bilddatensatz.

Alternativ können der medizinische Vergleichsbilddatensatz und der medizinische Bilddatensatz jeweils wenigstens einen Körperteil von verschiedenen Patienten abbilden. Dabei kann der medizinische Vergleichsbilddatensatz wenigstens einen Körperteil von dem ersten Patienten abbilden, während der medizinische Bilddatensatz wenigstens einen Körperteil von dem zweiten Patienten abbildet. Dabei sind der erste und der zweite Patient verschieden. Dabei kann der wenigstens eine abgebildete Körperteil in dem medizinischen Vergleichsbilddatensatz und dem medizinischen Bilddatensatz verschieden sein. Alternativ kann der medizinische Vergleichsbilddatensatz und der medizinische Bilddatensatz den gleichen wenigstens einen Körperteil von zwei verschiedenen Patienten abbilden.

Im Folgenden bedeutet die Formulierung "der medizinische Bilddatensatz betrifft den ersten oder zweiten Patienten", dass der medizinische Bilddatensatz wenigstens einen Körperteil des ersten oder zweiten Patienten abbildet. Analog bedeutet die Formulierung "der medizinische Vergleichsbilddatensatz betrifft den ersten Patienten", dass der medizinische Vergleichsbilddatensatz wenigstens einen Körperteil des ersten Patienten abbildet.

Die Erfinder haben erkannt, dass insbesondere ein medizinisches Bild wenigstens eines Körperteils eines Patienten dazu geeignet ist, biometrische Daten bzw. biometrischen Vergleichsdaten zu extrahieren. Mit anderen Worten haben die Erfinder erkannt, dass insbesondere basierend auf einem medizinischen Bild wenigstens eines Körperteils die biometrischen Daten bzw. biometrischen Vergleichsdaten des Patienten, dessen Körperteil abgebildet ist, bestimmt werden können. Die biometrischen Daten bzw. biometrischen Vergleichsdaten werden dabei durch den wenigstens einen in dem medizinischen Bilddatensatz bzw. medizinischen Vergleichsbilddatensatz abgebildeten Körperteil vorgegeben bzw. definiert bzw. bestimmt.

Nach einem weiteren Aspekt der Erfindung umfassten die biometrischen Daten wenigstens eine anatomische Landmarke und/oder wenigstens ein Oberflächennetz wenigstens einer Anatomie des abgebildeten Körperteils in den medizinischen Bilddaten. Dabei umfassen die biometrischen Vergleichsdaten wenigstens eine anatomische Landmarke und/oder wenigstens ein Oberflächennetz wenigstens einer Anatomie des abgebildeten Körperteils in dem medizinischen Vergleichsbilddatensatz.

Die wenigstens eine anatomische Landmarke gibt dabei eine Position einer Anatomie in dem entsprechenden Bilddatensatz insbesondere in dem medizinischen Bilddatensatz bzw. in dem medizinischen Vergleichsbilddatensatz an. Mit anderen Worten gibt die anatomische Landmarke die Position der Anatomie in dem von dem medizinischen Bilddatensatz bzw. dem medizinischen Vergleichsbilddatensatz umfassten medizinischen Bild an. Mit anderen Worten betrifft bzw. beschreibt die anatomische Landmarke die Anatomie. Die Anatomie, deren Position durch die anatomische Landmarke beschrieben wird, kann beispielsweise eine der folgenden Anatomien sein: Zentrum des Aortenbogens, Aortenwurzel, Verzweigung der Arteria brachiocephalica, Verzweigung der linken Arteria Subclavia und der Arteria Vertebralis, Verzweigung der rechten Arteria Subclavia und der Arteria Vertebralis, Truncus coeliacus, linke Halsschlagader, Carina Tracheae, Herz, obere Spitze der linken Niere, obere Spitze der rechten Niere, obere Spitze des linken Lungenflügels, obere Spitze des rechten Lungenflügels, Zentrum der Leber, obere Spitze der Leber, Bauchspeicheldrüse, Abgang der Nierenarterie, Spitze des Sternum, Wirbelkörper: C7, T1, T2, T3, T4, T5, T6, T7, T8, T9, T10, T11, T12, L1.

Die wenigstens eine anatomische Landmarke in dem medizinischen Vergleichsbilddatensatz bzw. in dem medizinischen Bilddatensatz kann beispielsweise wie in "Ghesu FC, Georgescu B, Zheng Y, Grbic S, Maier A, Hornegger J, Comaniciu D, "Multi-Scale Deep Reinforcement Learning for Real-Time 3D-Landmark Detection in CT Scans", IEEE Trans Pattern Anal Mach Intell. 2017" beschrieben, bestimmt worden sein.

Das wenigstens eine Oberflächennetz beschreibt eine Oberfläche einer Anatomie in dem medizinischen Bilddatensatz bzw. in dem medizinischen Vergleichsbilddatensatz. Mit anderen Worten beschreibt das wenigstens eine Oberflächennetz die Oberfläche der Anatomie in dem von dem medizinischen Bilddatensatz bzw. dem medizinischen Vergleichsbilddatensatz umfassten medizinischen Bild. Das Oberflächennetz kann dabei eine Mehrzahl an Punkten auf der Oberfläche der Anatomie in dem medizinischen Bild umfassen. Die Anatomie kann dabei beispielsweise der linke obere Lungenlappen, der linke untere Lungenlappen, der rechte obere Lungenlappen, der rechte mittlere Lungenlappen, der rechte untere Lungenlappen, das Herz, die Aorta, die Wirbelkörper sein. Das wenigstens eine Oberflächennetz kann basierend auf einer Segmentierung der Anatomie in dem medizinischen Bild bestimmt worden sein.

Die biometrischen Daten können insbesondere anatomische Landmarken von mehreren, verschiedenen Anatomien umfassen. Alternativ oder zusätzlich können die biometrischen Daten Oberflächennetze von mehr als einer Anatomie umfassen. Dabei können jeweils zwei anatomische Landmarken bzw. Oberflächennetze verschiedene Anatomien betreffen. Die biometrischen Vergleichsdaten können insbesondere anatomische Landmarken von mehreren, verschiedenen Anatomien umfassen. Alternativ oder zusätzlich können die biometrischen Vergleichsdaten Oberflächennetze von mehr als einer Anatomie umfassen. Dabei können jeweils zwei anatomische Landmarken bzw. Oberflächennetze verschiedene Anatomien betreffen.

Dabei kann die wenigstens eine anatomische Landmarke bzw. das wenigstens eine Oberflächennetz der biometrischen Daten dieselbe Anatomie betreffen, wie die wenigstens eine anatomische Landmarke bzw. das wenigstens eine Oberflächennetz der biometrischen Vergleichsdaten. Alternativ kann die wenigstens eine anatomische Landmarke bzw. das wenigstens eine Oberflächennetz der biometrischen Daten eine andere Anatomie betreffen als die wenigstens eine anatomische Landmarke bzw. das wenigstens eine Oberflächennetz der biometrischen Vergleichsdaten.

Wenn die biometrischen Daten bzw. die biometrischen Vergleichsdaten mehr als eine anatomische Landmarke und/oder mehr als ein Oberflächennetz umfassen, umfassen die biometrischen Daten bzw. die biometrischen Vergleichsdaten insbesondere auch eine Information über eine Lage der einzelnen anatomischen Landmarken und/oder Oberflächennetze zueinander.

Die Erfinder haben erkannt, dass die biometrischen Daten bzw. die biometrischen Vergleichsdaten des ersten oder zweiten Patienten durch wenigstens eine anatomische Landmarke bzw. wenigstens ein Oberflächennetz beschrieben werden können. Die Erfinder haben erkannt, dass die wenigstens eine anatomische Landmarke bzw. das wenigstens eine Oberflächennetz einmalige Eigenschaften des ersten bzw. zweiten Patienten abbilden können. Insbesondere haben die Erfinder erkannt, dass die Lage mehrerer anatomischer Landmarken bzw. Oberflächennetze zueinander die einmaligen Eigenschaften des ersten bzw. zweiten Patienten besonders gut abbilden. Mit anderen Worten kann die Lage der anatomischen Landmarken und/oder Oberflächennetze zueinander bzw. ihre räumliche Beziehung die Einzigartigkeit der biometrischen Daten bzw. der biometrischen Vergleichsdaten für den ersten bzw. zweiten Patienten begründen.

Nach einem weiteren Aspekt der Erfindung umfasst die anatomische Landmarke und/oder das Oberflächennetz wenigstens eine Koordinate in dem medizinischen Bilddatensatz bzw. in dem medizinischen Vergleichsbilddatensatz.

Die Koordinate beschreibt dabei, wo in dem von dem medizinischen Bilddatensatz bzw. dem medizinischen Vergleichsbilddatensatz umfassten medizinischen Bild die anatomische Landmarke und/oder das Oberflächennetz liegt. Dabei kann die Koordinate beispielsweise eine Koordinate des Pixels oder Voxels in dem entsprechenden medizinischen Bild angeben, der die anatomische Landmarke oder einen Punkt des Oberflächennetzes abbildet. Die Koordinate des Pixels ist dabei ein Wertepaar, das den Ort des Pixels in der Pixelmatrix definiert. Die Koordinate des Voxels ist dabei ein Tripel, das den Ort des Voxels in der Voxelmatrix definiert.

In Ausführungen der Erfindung kann beispielsweise der medizinische Bilddatensatz ein dreidimensionales medizinisches Bild umfassen und der medizinische Vergleichsbilddatensatz ein zweidimensionales medizinisches Bild. Der medizinische Vergleichsbilddatensatz umfasst als medizinisches Bild bzw. Abbildung des Patienten somit eine zweidimensionale Projektion in eine Abbildungsebene. Insbesondere umfassen dann die biometrischen Daten wenigstens ein Tripel, welches die Lage der wenigstens einen Landmarke in dem medizinischen Bilddatensatz definiert. Insbesondere umfassen dann die biometrischen Vergleichsdaten wenigstens ein Wertepaar, welches die Lage der wenigstens einen Landmarke in dem medizinischen Vergleichsbilddatensatz definiert. Um das Abweichungsmaß bestimmen zu können, können die biometrischen Daten in eine zweidimensionale Ebene projiziert werden. Dabei kann die zweidimensionale Ebene der Abbildungsebene der biometrischen Vergleichsdaten entsprechen. Alternativ kann der medizinische Vergleichsbilddatensatz ein dreidimensionales medizinisches Bild umfassen und der medizinische Bilddatensatz ein zweidimensionales medizinisches Bild. In diesem Fall können die biometrischen Vergleichsdaten in eine zweidimensionale Ebene projiziert werden.

Das Oberflächennetz kann dabei mehr als eine Koordinate umfassen. Die Koordinaten sind dabei auf einer Oberfläche der Anatomie angeordnet, die durch das Oberflächennetz beschrieben wird. Dabei definiert jede Koordinate eines Oberflächennetzes einen Punkt auf der Oberfläche der Anatomie, die durch das Oberflächennetz abgebildet bzw. dargestellt bzw. beschrieben wird.

Wenn die biometrischen Daten bzw. die biometrischen Vergleichsdaten mehr als eine anatomische Landmarke und/oder mehr als ein Oberflächennetz umfassen, kann von den Koordinaten der einzelnen anatomischen Landmarken und/oder der Oberflächennetze auf eine Lage der anatomischen Landmarken und/oder Oberflächennetze zueinander geschlossen werden. Die Lage zueinander beschreibt eine räumliche Beziehung der anatomischen Landmarken und/oder Oberflächennetze. Die räumliche Beziehung kann dabei die Abstände zwischen den einzelnen anatomischen Landmarken und/oder Oberflächennetzen und/oder die räumliche Anordnung der anatomischen Landmarken und/oder der Oberflächennetze im zwei- oder dreidimensionalen Raum beschreiben. Insbesondere kann die räumliche Beziehung auch eine Form eines Oberflächennetzes beschreiben. Die Form wird dabei von der Form der Anatomie, die das Oberflächennetz beschreibt, vorgegeben.

Durch Kenntnis eines Maßstabes des abgebildeten Körperteils in den medizinischen Bilddaten bzw. den medizinischen Vergleichsbilddaten kann auf einen Abstand zwischen einzelnen anatomischen Landmarken und/oder Punkten der Oberflächennetze in einer metrischen Einheit geschlossen werden. Eine metrische Einheit kann beispielsweise Millimeter, Zentimeter, Meter. Alternativ kann der Abstand in einer alternativen Abstandseinheit beispielsweise in Inch oder Foot angegeben werden. Die räumliche Beziehung kann dabei die Lage der anatomischen Landmarken und/oder Oberflächennetze zueinander im Raum definieren.

In Ausführungen der Erfindung kann die Koordinate eine Lage der anatomischen Landmarke bzw. eines Punktes des Oberflächennetzes relativ zu dem abgebildeten Körperteil angeben. Beispielsweise kann ein "Nullpunkt" in dem abgebildeten Körperteil vorgegeben sein. Der Nullpunkt kann dabei dem Ursprung des Koordinatensystems entsprechen, in dem die Koordinaten angegeben sind. Der Nullpunkt kann beispielsweise an einem markanten Punkt angeordnet sein. Der Nullpunkt kann beispielsweise der Schwertfortsatz oder das Zentrum des Herzens oder die Verzweigung der Trachea etc. sein. Der Nullpunkt kann insbesondere eine anatomische Landmarke sein. Die Koordinate der anatomischen Landmarke bzw. der Oberflächennetzes kann dann in Relation zu dem Nullpunkt angeben sein. Dabei können die Achsen des Koordinatensystems parallel zu den Körperachsen des Patienten angeordnet sein. Die Körperachsen sind insbesondere cranio-caudal, dorsal-frontal bzw. posterior-anterior und lateral-medial. Die Koordinate kann dabei den Abstand der anatomischen Landmarker bzw. des Punktes des Oberflächennetzes zum Ursprung in metrischen Einheiten bzw. einer alternativen Abstandseinheit angeben.

Die Erfinder haben erkannt, dass anhand von Koordinaten Lagebeziehungen bzw. räumliche Beziehungen der anatomischen Landmarken und/oder Oberflächennetze beschrieben werden können. Außerdem habend die Erfinder erkannt, dass basierend auf den Koordinaten Abstände zwischen einzelnen anatomischen Landmarkern bzw. Punkten eines Oberflächennetzes bestimmt werden können. Die Erfinder haben erkannt, dass auf diese Weise eine Generalisierung in ein einheitliches Abstandsmaß bzw. eine einheitliche Abstandseinheit beispielsweise eine metrische Einheit möglich ist. Die Erfinder haben erkannt, dass ein Koordinatensystem zugrunde gelegt werden kann, dessen Position des Ursprungs an eine Position des ersten bzw. zweiten Patienten in dem medizinischen Bilddatensatz bzw. in dem medizinischen Vergleichsbilddatensatz angepasst ist. Die Erfinder haben erkannt, dass mittels einer Projektion zweidimensionale und dreidimensionale biometrische Daten verglichen werden können.

Nach einem weiteren Aspekt der Erfindung umfasst das Verfahren einen Verfahrensschritt eines Herstellens einer Registrierung zwischen den biometrischen Vergleichsdaten und den biometrischen Daten. Dabei erfolgt der Verfahrensschritt des Bestimmens des Abweichungsmaßes basierend auf der Registrierung.

In dem Verfahrensschritt der Herstellung der Registrierung werden insbesondere die anatomischen Landmarken und/oder Oberflächennetze der biometrischen Daten und der biometrischen Vergleichsdaten in eine räumliche Beziehung zueinander gesetzt. Insbesondere können die biometrischen Daten und die biometrischen Vergleichsdaten relativ räumlich zueinander positioniert werden.

Insbesondere können die biometrischen Daten mehr als eine anatomische Landmarke und/oder mehr als ein Oberflächennetz umfassen. Dabei betreffen jeweils zwei anatomische Landmarken bzw. Oberflächennetze verschiedene Anatomien. Insbesondere können die biometrischen Vergleichsdaten mehr als eine anatomische Landmarke und/oder mehr als ein Oberflächennetz umfassen. Dabei betreffen jeweils zwei anatomische Landmarken bzw. Oberflächennetze verschiedene Anatomien. Insbesondere können dabei die anatomischen Landmarken bzw. Oberflächennetze der biometrischen Daten und die biometrischen Landmarken bzw. Oberflächennetze der biometrischen Vergleichsdaten wenigstens teilweise dieselben Anatomien betreffen bzw. beschreiben. Mit anderen Worten kann wenigstens eine anatomische Landmarke bzw. Oberflächennetz der biometrischen Daten dieselbe Anatomie betreffen, wie eine anatomische Landmarke bzw. ein Oberflächennetz der biometrischen Vergleichsdaten. Beim Registrieren kann insbesondere die räumliche Beziehung der anatomischen Landmarken und/oder Oberflächennetze der biometrischen Daten und die räumliche Beziehung der anatomischen Landmarken und/oder Oberflächennetze der biometrischen Vergleichsdaten beibehalten werden bzw. unverändert bleiben. Durch das Registrieren werden die anatomischen Landmarken und/oder Oberflächennetze der biometrischen Daten relativ zu den anatomischen Landmarken und/oder Oberflächennetzen der biometrischen Vergleichsdaten positioniert.

Das Abweichungsmaß vergleicht die Position jeweils einer anatomischen Landmarke der biometrischen Daten und einer anatomischen Landmarke der biometrischen Vergleichsdaten, die dieselbe Anatomie betreffen. Alternativ oder zusätzlich vergleicht das Abweichungsmaß die Form und/oder Position eines Oberflächennetzes der biometrischen Daten mit der Form und/oder Position eines Oberflächennetzes der biometrischen Vergleichsdaten, die dieselbe Anatomie betreffen. Insbesondere vergleicht das Abweichungsmaß die Position und/oder Form aller einander entsprechenden anatomischen Landmarken und/oder Oberflächennetze der biometrischen Daten und der biometrischen Vergleichsdaten. Einander entsprechende anatomische Landmarken und/oder Oberflächennetze betreffen dabei dieselbe Anatomie.

Die Erfinder haben erkannt, dass basierend auf einer Registrierung ein Vergleich der Positionen der anatomischen Landmarken und/oder Oberflächennetzen der biometrischen Daten und der biometrischen Vergleichsdaten möglich ist. Die Erfinder haben erkannt, dass die räumliche Beziehung bzw. Lage bzw. Anordnung der anatomischen Landmarken und/oder Oberflächennetze einmalig für einen bestimmten Patienten ist. Die Erfinder haben erkannt, dass es möglich ist, basierend auf der Registrierung ein Abweichungsmaß zu ermitteln bzw. zu bestimmen, welches angibt wie stark die Positionen und damit die räumliche Beziehung der anatomischen Landmarken und/oder Oberflächennetze der biometrischen Daten von denen der biometrischen Vergleichsdaten abweicht.

Nach einem weiteren Aspekt der Erfindung umfasst der Verfahrensschritt des Herstellens der Registrierung einen Verfahrensschritt eines Bestimmens von einander entsprechenden anatomischen Landmarken und/oder Oberflächennetzen in den biometrischen Vergleichsdaten und den biometrischen Daten und einen Verfahrensschritt eines Transformierens der einander anatomischen entsprechenden Landmarken und/oder Oberflächennetze in ein gemeinsames Koordinatensystem. Dabei umfasst der Verfahrensschritt des Bestimmens des Abweichungsmaßes einen Verfahrensschritt eines Bestimmens einer Abweichung der Koordinaten der einander entsprechenden anatomischen Landmarken bzw. Oberflächennetze in dem gemeinsamen Koordinatensystem.

In dem Verfahrensschritt des Bestimmens von einander entsprechenden anatomischen Landmarken und/oder Oberflächennetzen wird wenigstens eine anatomische Landmarke und/oder Oberflächennetz der biometrischen Daten einer anatomischen Landmarke und/oder Oberflächennetz der biometrischen Vergleichsdaten zugeordnet, die dieselbe Anatomie betrifft bzw. deren Lage bzw. Position beschreibt. "Entsprechend" bedeutet in diesem Fall, dass die einander entsprechenden anatomischen Landmarken bzw. Oberflächennetze dieselbe Anatomie betreffen.

Wenn die biometrischen Daten und/oder die biometrischen Vergleichsdaten mehr als eine anatomische Landmarke und/oder Oberflächennetz umfassen, können die anatomischen Landmarken und/oder Oberflächennetze der biometrischen Daten und der biometrischen Vergleichsdaten wenigstens teilweise paarweise einander zugeordnet werden. Dabei werden einander entsprechende also dieselbe Anatomie betreffende anatomische Landmarken und/oder Oberflächennetze einander zugeordnet.

In Ausführungen kann nicht jeder anatomischen Landmarke und/oder Oberflächennetz eine entsprechende anatomische Landmarke und/oder Oberflächennetz zugordnet werden. Diese anatomischen Landmarken und/oder Oberflächennetze können beim Bestimmen des Abweichungsmaßes vernachlässigt bzw. nicht berücksichtigt werden.

In dem Verfahrensschritt des Transformierens der einander entsprechenden Landmarken und/oder Oberflächennetze in ein gemeinsames Koordinatensystem wird die wenigstens eine anatomische Landmarke und/oder das wenigstens eine Oberflächennetz der biometrischen Daten in ein gemeinsames Koordinatensystem mit der wenigstens einen anatomischen Landmarke und/oder dem wenigstens einen Oberflächennetz der biometrischen Vergleichsdaten überführt.

Wenn die biometrischen Daten und/oder die biometrischen Vergleichsdaten mehr als eine anatomische Landmarke und/oder mehr als ein Oberflächennetz umfassen, können wenigstens die anatomischen Landmarken und/oder Oberflächennetze der biometrischen Daten in das gemeinsame Koordinatensystem transformiert werden, die ein entsprechendes Gegenstück in den biometrischen Vergleichsdaten haben. Analog können wenigstens die anatomischen Landmarken und/oder Oberflächennetze der biometrischen Vergleichsdaten in das gemeinsame Koordinatensystem transformiert werden, die ein entsprechendes Gegenstück in den biometrischen Daten haben. Alternativ können alle anatomischen Landmarken und/oder Oberflächennetze der biometrischen Daten und der biometrischen Vergleichsdaten in das gemeinsame Koordinatensystem transformiert werden.

Insbesondere können beim Transformieren in ein gemeinsames Koordinatensystem die anatomischen Landmarken und/oder Oberflächennetze der biometrischen Daten die räumliche Beziehung untereinander beibehalten, wenn die biometrischen Daten mehr als eine anatomische Landmarke und/oder mehr als ein Oberflächennetz umfassen. Analog können die anatomischen Landmarken und/oder Oberflächennetze der biometrischen Vergleichsdaten die räumliche Beziehung untereinander beibehalten, wenn die biometrischen Vergleichsdaten mehr als eine anatomische Landmarke und/oder mehr als ein Oberflächennetz umfassen.

Das gemeinsame Koordinatensystem kann dabei die räumliche Lage bzw. Verteilung der anatomischen Landmarken und/oder Oberflächennetze zueinander in einem metrischen System abbilden. Alternativ kann das gemeinsame Koordinatensystem die räumliche Lage bzw. Verteilung in einem alternativen Abstandssystem beispielsweise dem angloamerikanischen Maßsystem abbilden.

Insbesondere kann in dem gemeinsamen Koordinatensystem die wenigstens eine anatomische Landmarke und/oder Oberflächennetz der biometrischen Daten relativ zu der wenigstens einen Landmarke und/oder Oberflächennetz der biometrischen Vergleichsdaten positioniert werden.

Beim Transformieren kann insbesondere die räumliche Beziehung bzw. Lage bzw. Anordnung der anatomischen Landmarken und/oder Oberflächennetze der biometrischen Daten an einen Maßstab des gemeinsamen Koordinatensystems angepasst werden. Analog kann die räumliche Lage bzw. Anordnung der anatomischen Landmarken und/oder Oberflächennetze der biometrischen Vergleichsdaten an den Maßstab des gemeinsamen Koordinatensystems angepasst werden. Der Maßstab kann insbesondere ein Abstandsmaß bzw. Streckenmaß vorgeben. Der Maßstab wird dabei insbesondere durch das System bzw. Maßsystem des gemeinsamen Koordinatensystems vorgegeben. Das Abstandsmaß kann beispielsweise in metrischen Einheiten oder in dem angloamerikanischen Maßsystem angegeben werden.

Beim Transformieren kann wenigstens eine anatomische Landmarke der biometrischen Daten auf eine entsprechende anatomische Landmarke der biometrischen Vergleichsdaten in dem gemeinsamen Koordinatensystem abgebildet werden. Insbesondere bedeutet dies, dass die beiden Landmarken, wenn sie einander entsprechen, beim Transformieren in einem gemeinsamen Koordinatensystem an dieselbe Position gesetzt werden. Alternativ kann ein Oberflächennetz der biometrischen Daten auf ein Oberflächennetz der biometrischen Vergleichsdaten abgebildet werden. Alle anderen anatomischen Landmarken und/oder Oberflächennetze der biometrischen Daten und der biometrischen Vergleichsdaten werden dann der räumlichen Beziehung entsprechend relativ zu der wenigstens einen anatomischen Landmarke oder Oberflächennetz in dem gemeinsamen Koordinatensystem positioniert.

Beispielsweise können die beiden oben beschriebenen Nullpunkte der biometrischen Daten und der biometrischen Vergleichsdaten aufeinander abgebildet bzw. an dieselbe Position in dem gemeinsamen Koordinatensystem gesetzt werden, wenn die beiden Nullpunkte einander entsprechende anatomische Landmarken betreffen.

Insbesondere kann ein Paar anatomischer Landmarken oder Oberflächennetze als Bezugspunkt bzw. Nullpunkt für die Transformation in ein gemeinsames Koordinatensystem genutzt werden.

Insbesondere kann dieses Paar an eine gemeinsame Position in dem gemeinsamen Koordinatensystem, beispielsweise den Ursprung, positioniert werden. Alle anderen anatomischen Landmarken und/oder Oberflächennetze können ihre räumliche Beziehung relativ zu dem Paar beibehalten. Die räumliche Beziehung kann dabei an den Maßstab des gemeinsamen Koordinatensystems angepasst sein.

Alternativ können, wenn die biometrischen Daten und die biometrischen Vergleichsdaten jeweils mehr als eine anatomische Landmarke und/oder mehr als ein Oberflächennetz umfassen, die anatomischen Landmarken und/oder Oberflächennetze der biometrischen Daten unter Beibehaltung der räumlichen Beziehung relativ zu den anatomischen Landmarken und/oder Oberflächennetzen der biometrischen Vergleichsdaten derart in dem gemeinsamen Koordinatensystem positioniert werden, dass die Abstände der Positionen der jeweiligen entsprechenden anatomischen Landmarken und/oder Oberflächennetze minimiert werden. Insbesondere kann die Summe oder die quadratische Summe der Abstände minimiert werden.

In dem Verfahrensschritt des Bestimmens einer Abweichung der Koordinaten der einander entsprechenden Landmarken bzw. Oberflächennetze wird die Abweichung der Positionen der einander entsprechenden anatomischen Landmarken bzw. Oberflächennetze in dem gemeinsamen Koordinatensystem bestimmt. Insbesondere kann für jedes Paar von anatomischen Landmarken ein räumlicher Abstand der Positionen in dem gemeinsamen Koordinatensystem bestimmt werden. Das Abweichungsmaß kann dabei der Summe der Abstände der einzelnen Paare entsprechen. Alternativ kann das Abweichungsmaß der Wurzel aus der quadratischen Summe der Abstände der einzelnen Paare entsprechen. Wenn die Abstände der Koordinaten von mehr als einem Paar von anatomischen Landmarken und/oder Oberflächennetzen bestimmt werden, kann das wie oben beschrieben ausgebildete Abweichungsmaß durch eine Division durch die Anzahl der Paare normalisiert werden.

Bei einander entsprechenden Oberflächennetzen können die Abweichungen der Koordinaten bzw. Punkte, die die Oberflächennetze definieren, bestimmt werden. Alternativ kann jedes Oberflächennetz eine interpolierte Fläche zwischen den Koordinaten bzw. Punkten des Oberflächennetzes umfassen. Insbesondere kann dann die Abweichung der Koordinaten von zwei Oberflächennetzen eine Abweichung der Flächen in dem gemeinsamen Koordinatensystem umfassen.

Die Erfinder haben erkannt, dass eine Registrierung einen Vergleich der biometrischen Daten und der biometrischen Vergleichsdaten ermöglicht. Die Erfinder haben erkannt, dass, wenn die Registrierung eine Transformation in ein gemeinsames Koordinatensystem umfasst, ein Vergleich der räumlichen Beziehung der von den biometrischen Daten umfassten wenigstens einen anatomischen Landmarke und/oder Oberflächennetz mit der räumlichen Beziehung der von den biometrischen Vergleichsdaten umfassten wenigstens einen anatomischen Landmarke und/oder Oberflächennetz ermöglicht. Die Erfinder haben erkannt, dass das derart bestimmt Abweichungsmaß angibt, wie gut die räumliche Beziehung der anatomischen Landmarken und/oder Oberflächennetze der biometrischen Daten mit der räumlichen Beziehung der anatomischen Landmarken und/oder Oberflächennetze der biometrischen Vergleichsdaten übereinstimmt.

Nach einem weiteren Aspekt der Erfindung umfasst das Herstellen der Registrierung der biometrischen Vergleichsdaten und der biometrischen Daten eine rigide Registrierung.

Mit anderen Worten ist die Registrierung eine rigide Registrierung.

Insbesondere umfassen die biometrischen Daten und die biometrischen Vergleichsdaten jeweils mehr als eine anatomische Landmarke und/oder mehr als ein Oberflächennetz. Insbesondere stehen die anatomischen Landmarken und/oder Oberflächennetze der biometrischen Daten bzw. der biometrischen Vergleichsdaten in einer räumlichen Beziehung bzw. Lage bzw. Anordnung zueinander. Mit anderen Worten weisen die anatomischen Landmarken und/oder Oberflächennetze der biometrischen Daten bzw. biometrischen Vergleichsdaten definierte Abstände zueinander auf. Die Abstände können dabei in metrischen Einheiten oder im angloamerikanischen Maßsystem angegeben sein. Die räumliche Beziehung kann auch beschreiben, wie die anatomischen Landmarken und/oder Oberflächennetze in einem zweidimensionalen oder dreidimensionalen Raum relativ zueinander angeordnet sind.

Bei der rigiden Registrierung wird die räumliche Beziehung der anatomischen Landmarken und/oder Oberflächennetzen der biometrischen Daten beibehalten. Analog wird bei der rigiden Registrierung die räumliche Beziehung der anatomischen Landmarken und/oder Oberflächennetze der biometrischen Vergleichsdaten beibehalten. Bei der rigiden Registrierung werden lediglich die Abstände zwischen den anatomischen Landmarken und/oder Oberflächennetzen der biometrischen Daten bzw. der biometrischen Vergleichsdaten relativ zueinander, das heißt alle Abstände gleichermaßen verändert.

Bei einer Ausführung der rigiden Registrierung wird somit eine anatomische Landmarke oder ein Oberflächennetz der biometrischen Daten auf die entsprechende anatomische Landmarke oder das entsprechende Oberflächennetz der biometrischen Vergleichsdaten abgebildet. Mit anderen Worten wird eine anatomische Landmarke oder ein Oberflächennetz der biometrischen Daten an dieselbe Position in dem gemeinsamen Koordinatensystem positioniert wie die entsprechende anatomische Landmarke oder das entsprechende Oberflächennetz der biometrischen Vergleichsdaten. Alle anderen anatomischen Landmarken oder Oberflächennetze werden anhand ihrer räumlichen Beziehung zueinander in dem gemeinsamen Koordinatensystem positioniert. Lediglich die Abstände können an den Maßstab des gemeinsamen Koordinatensystems angepasst werden.

Bei einer alternativen Ausführung der rigiden Registrierung werden die anatomischen Landmarken und/oder Oberflächennetze wie oben beschrieben unter Beibehaltung der räumlichen Beziehung derart in dem gemeinsamen Koordinatensystem positioniert, dass die Abstände zwischen den einander entsprechenden anatomischen Landmarken und/oder Oberflächennetzen minimiert werden. "Beibehalten der räumlichen Beziehung" bedeutet auch hier, dass lediglich die Abstände zwischen den anatomischen Landmarken und/oder Oberflächennetzen an den Maßstab des Koordinatensystems angepasst werden. Die absoluten Abstände in einer metrischen bzw. anglo-amerikanischen Einheit werden beibehalten. Ebenso wird die räumliche Anordnung im zwei- oder dreidimensionalen Raum beibehalten.

Die Erfinder haben erkannt, dass eine spezifische räumliche Beziehung der anatomischen Landmarken und/oder Oberflächennetze für einen Patienten einmalig ist. Die Erfinder haben erkannt, dass diese räumliche Beziehung bei einer rigiden Registrierung beibehalten wird. Die Erfinder haben erkannt, dass auf diese Weise festgestellt werden kann, ob auf Grund der räumlichen Beziehung davon ausgegangen werden kann, dass die biometrischen Daten von einem medizinischen Bild desselben Patienten stammen, wie die biometrischen Vergleichsdaten oder nicht.

Nach einem weiteren Aspekt der Erfindung umfasst der Verfahrensschritt des Bestimmens des Abweichungsmaßes einen Verfahrensschritt eines Anwendens einer trainierten Funktion auf die biometrischen Vergleichsdaten und die biometrischen Daten, wodurch das Abweichungsmaß bestimmt wird.

Im Allgemeinen ahmt eine trainierte Funktion kognitive Funktionen nach, die Menschen mit menschlichem Denken verbinden. Insbesondere durch auf Trainingsdaten basierendem Training kann sich die trainierte Funktion an neue Umstände anpassen sowie Muster erkennen und extrapolieren.

Im Allgemeinen können Parameter einer trainierten Funktion mittels Trainings angepasst werden. Insbesondere kann dafür ein beaufsichtigtes (supervised) Training, ein halbüberwachtes (semi-supervised) Training, ein unbeaufsichtigtes (unsupervised) Training, ein verstärkendes Lernen (reinforcement learning) und/oder ein aktives Lernen (active learning) verwendet werden. Darüber hinaus kann Repräsentationslernen (ein alternativer Begriff ist "Merkmalslernen") (representation learning bzw. feature learning) verwendet werden. Insbesondere können die Parameter der trainierten Funktionen durch mehrere Trainingsschritte iterativ angepasst werden.

Insbesondere kann eine trainierte Funktion ein neuronales Netzwerk, eine Unterstützungsvektormaschine (support vector machine), einen Zufallsbaum bzw. einen Entscheidungsbaum (decision tree) und/oder ein Bayes'sches Netzwerk umfassen, und/oder die trainierte Funktion kann auf k-Mittel-Clustering (k-means clustering), Q-Learning, genetischen Algorithmen und/oder Assoziationsregeln basieren. Insbesondere kann eine trainierte Funktion eine Kombination aus mehreren unkorrelierten Entscheidungsbäumen bzw. ein Ensemble aus Entscheidungsbäumen (random forest) umfassen. Insbesondere kann die trainierte Funktion mittels XGBoosting (extreme Gradient Boosting) bestimmt werden. Insbesondere kann ein neuronales Netzwerk ein tiefes neuronales Netzwerk (deep neural network), ein Faltungs-neuronales Netzwerk (convolutional neural network) oder ein Faltungs-tiefes neuronales Netzwerk (convolutional deep neural network) sein. Darüber hinaus kann ein neuronales Netzwerk ein kontradiktorisches Netzwerk (adversarial network), ein tiefes kontradiktorisches Netzwerk (deep adversarial network) und/oder ein generatives kontradiktorisches Netzwerk (generative adversarial network) sein. Insbesondere kann ein neuronales Netzwerk ein rekurrentes neuronales Netzwerk (recurrent neural network) sein. Insbesondere kann ein rekurrentes neuronales Netzwerk ein Netzwerk mit langem Kurzzeitgedächtnis (long-short-term-memory, LSTM), insbesondere eine Gated Recurrent Unit (GRU), sein. Insbesondere kann eine trainierte Funktion eine Kombination der beschriebenen Ansätze umfassen. Insbesondere werden die hier beschriebenen Ansätze für eine trainierte Funktion Netzwerkarchitektur der trainierten Funktion genannt.

Die trainierte Funktion kann somit auf die wenigstens eine anatomische Landmarke und/oder das wenigstens eine Oberflächennetz der biometrischen Daten und die wenigstens eine anatomische Landmarke und das wenigstens eine Oberflächennetz der biometrischen Vergleichsdaten angewendet werden. Dabei wird das Abweichungsmaß bestimmt. Das Abweichungsmaß gibt dabei an, wie stark die biometrischen Daten von den biometrischen Vergleichsdaten abweichen. Wenn die biometrischen Daten bzw. die biometrischen Vergleichsdaten mehr als eine anatomische Landmarke und/oder mehr als ein Oberflächennetz umfassen, gibt das Abweichungsmaß an, wie stark die räumliche Beziehung der anatomischen Landmarken und/oder der Oberflächennetze der biometrischen Daten von der räumlichen Beziehung der anatomischen Landmarken und/oder Oberflächennetze der biometrischen Vergleichsdaten abweicht. Die räumliche Beziehung kann dabei wie oben beschrieben die Abstände zwischen den einzelnen anatomischen Landmarken und/oder Oberflächennetzen und/oder die räumliche Anordnung der anatomischen Landmarken und/oder der Oberflächennetze im zwei- oder dreiDimensionalen beschreiben. Insbesondere kann die räumliche Beziehung auch eine Form eines Oberflächennetzes beschreiben. Die Form wird dabei von der Form der Anatomie, die das Oberflächennetz beschreibt, vorgegeben. Insbesondere kann somit das Abweichungsmaß eine Abweichung der Positionen der anatomischen Landmarken und/oder Oberflächennetze der biometrischen Daten und der anatomischen Landmarken und/oder Oberflächennetze der biometrischen Vergleichsdaten beschreiben. Dabei werden insbesondere wie oben beschrieben die Positionen von einander entsprechenden anatomischen Landmarken und/oder Oberflächennetzen verglichen.

Das Abweichungsmaß kann dabei angeben, wie wahrscheinlich die biometrischen Daten und die biometrischen Vergleichsdaten von unterschiedlichen Patienten stammen. Mit anderen Worten kann das Abweichungsmaß angeben, wie wahrscheinlich der medizinische Bilddatensatz den wenigstens einen Körperteil des zweiten Patienten abbildet, wenn der medizinische Vergleichsbilddatensatz den wenigstens einen Körperteil des ersten Patienten abbildet. Je größer das Abweichungsmaß, desto wahrscheinlicher ist es, dass der medizinische Bilddatensatz einen anderen Patienten abbildet als der medizinische Vergleichsbilddatensatz.

Die trainierte Funktion kann mit einer Mehrzahl von biometrischen Trainingsdaten und biometrischen Trainingsvergleichsdaten trainiert werden. Dabei wird die trainierte Funktion auf jeweils ein Paar von biometrischen Trainingsdaten und biometrischen Trainingsvergleichsdaten angewendet. Dabei wird ein Trainingsabweichungsmaß bestimmt. Außerdem wird im Vorfeld ein manuelles Abweichungsmaß zwischen den biometrischen Trainingsdaten und den biometrischen Trainingsvergleichsdaten bestimmt. Das Trainingsabweichungsmaß wird mit dem manuell bestimmten Abweichungsmaß verglichen. Die Parameter der trainierten Funktion werden dann derart angepasst, dass bei einem erneuten Anwenden das Trainingsabweichungsmaß möglichst gut mit dem manuell bestimmten Abweichungsmaß übereinstimmt. Das manuell bestimmte Abweichungsmaß kann dabei insbesondere von einem Experten bestimmt werden.

Die Erfinder haben erkannt, dass das Abweichungsmaß mit Hilfe einer trainierten Funktion bestimmbar ist. Die Erfinder haben erkannt, dass auf diese Weise auf eine Registrierung verzichtet werden kann. Die Erfinder haben erkannt, dass Fehler, die bei der Transformation der biometrischen Daten und der biometrischen Vergleichsdaten in das gemeinsame Koordinatensystem auftreten können, reduziert werden können, wenn das Abweichungsmaß mit der trainieren Funktion bestimmt wird.

Nach einem weiteren Aspekt der Erfindung hängt ein Einfluss der wenigstens einen anatomischen Landmarke und/oder des wenigstens einen Oberflächennetzes auf das Abweichungsmaß von ihrer zeitlichen Stabilität ab.

Der Einfluss beschreibt dabei eine Wichtung, mit der die wenigstens eine anatomische Landmarke und/oder das wenigstens eine Oberflächennetz beim Bestimmen des Abweichungsmaßes berücksichtigt wird. Der Einfluss einer anatomischen Landmarke bzw. einer Oberfläche kann beispielsweise durch eine Wichtung beispielsweise der Position der anatomischen Landmarke bzw. des Oberflächennetzes in dem gemeinsamen Koordinatensystem beim Bestimmen des Abweichungsmaßes definiert sein.

Beispielsweise kann die Abweichung der Koordinaten von zwei einander entsprechenden anatomischen Landmarken und/oder Oberflächennetzen in dem gemeinsamen Koordinatensystem beim Bestimmen des Abweichungsmaßes gewichtet werden. Die Wichtung bestimmt dabei den Einfluss der anatomischen Landmarken und/oder des Oberflächennetzes auf das Abweichungsmaß. Wenn die biometrischen Daten bzw. die biometrischen Vergleichsdaten mehr als eine anatomische Landmarke und/oder mehr als ein Oberflächennetz umfassen, kann die Abweichung der Koordinaten von zwei einander entsprechenden Landmarken bzw. Oberflächennetzen in Abhängigkeit ihrer zeitlichen Stabilität beim Bestimmen des Abweichungsmaßes gewichtet werden. Dafür kann die Abweichung der Koordinaten mit einem Wichtungsfaktor multipliziert werden. Der Wichtungsfaktor kann für unterschiedliche anatomische Landmarken bzw. Oberflächennetze verschieden sein. Das Abweichungsmaß kann dann die Summe oder die quadratische Summe oder die Wurzel der quadratischen Summe der derart gewichteten Abweichungen aller einander entsprechenden Landmarken und/oder Oberflächennetze sein.

Alternativ kann eine Wichtung der einzelnen anatomischen Landmarken und/oder Oberflächennetze in der trainierten Funktion von der zeitlichen Stabilität abhängen. Die trainierte Funktion kann beim Bestimmen des Abweichungsmaßes zeitlich stabile anatomische Landmarken und/oder Oberflächennetze stärker gewichten als zeitlich instabile anatomische Landmarken und Oberflächennetze. Eine größere Gewichtung bedeutet dabei einen größeren Einfluss auf das Abweichungsmaß.

Zeitlich stabil bedeutet dabei, dass sich die anatomische Landmarke bzw. das Oberflächennetz mit der Zeit nicht oder wenig verändert. Eine zeitlich stabile anatomische Landmarke kann beispielsweise ein Knochen und/oder ein Wirbelkörper und/oder der Schwertfortsatz etc. betreffen. Bei erwachsenen Patienten ohne Erkrankung der Knochen wie beispielsweise Osteoporose sind derartige anatomische Landmarken räumlich und zeitlich nahezu unveränderlich.

Eine zeitlich nicht stabile anatomische Landmarke kann beispielsweise eine untere Lungenspitze betreffen. Diese ändert im Laufe eines Atemzugs permanent ihre Position relativ zu einer zeitlich stabilen anatomischen Landmarke. Das heißt, dass eine Abweichung der Position bzw. der Koordinaten der unteren Lungenspitze zwischen den biometrischen Daten und den biometrischen Vergleichsdaten bedeuten kann, dass es sich um unterschiedliche Patienten handelt. Alternativ kann allerdings auch derselbe Patient zu unterschiedlichen Zeitpunkten eines Atemintervalls in dem medizinischen Bilddatensatz und dem medizinischen Vergleichsbilddatensatz erfasst worden sein.

Analog gilt dies auch für Oberflächennetze. Ein zeitlich stabiles Oberflächennetz kann beispielsweise eine Oberfläche eines Sternums beschreiben. Ein zeitlich instabiles Oberflächennetz kann beispielsweise eine Oberfläche eines Lungenlappens oder des Herzens sein.

Die Erfinder haben erkannt, dass eine Abweichung von zeitlich instabilen anatomischen Landmarken und/oder Oberflächennetzen eine geringere Aussagekraft bezüglich der biometrischen Eigenschaften eines Patienten hat als zeitlich stabile anatomische Landmarken und/oder Oberflächennetze. Die Erfinder haben erkannt, dass dies durch eine Wichtung beim Bestimmen des Abweichungsmaßes berücksichtigt werden kann. Mit anderen Worten kann der Einfluss der wenigstens einen anatomischen Landmarke und/oder des wenigstens einen Oberflächennetzes der biometrischen Daten bzw. der biometrischen Vergleichsdaten auf das Abweichungsmaß durch eine Wichtung der Abweichung der Koordinaten oder durch eine Wichtung der wenigstens einen anatomischen Landmarke und/oder Oberflächennetz in der trainierten Funktion berücksichtigt werden.

Nach einem optionalen Aspekt der Erfindung umfasst das Verfahren einen Verfahrensschritt eines Klassifizierens der wenigstens einen anatomischen Landmarke und/oder des wenigstens einen Oberflächennetzes nach ihrer zeitlichen Stabilität. Dabei hängt der Einfluss der wenigstens einen anatomischen Landmarke und/oder des wenigstens einen Oberflächennetzes auf das Abweichungsmaß von der Klassifikation ab. Dabei basiert das Klassifizieren auf einem bekannten anatomisch begründeten Verhalten der wenigstens einen anatomischen Landmarke und/oder des wenigstens einen Oberflächennetzes.

Das bekannte anatomisch begründete Verhalten ist insbesondere einem Fachmann bekannt. Der Fachmann kennt die zeitliche Stabilität von verschiedenen Anatomien. Anatomisch begründet bedeutet dabei, dass die zeitliche Stabilität bzw. das Verhalten allgemein gültig ist. Mit anderen Worten ist die zeitliche Stabilität durch die Anatomie begründet. Insbesondere kann die zeitliche Stabilität der wenigstens einen anatomischen Landmarker und/oder des wenigstens einen Oberflächennetzes also beispielsweise durch die "Aufgabe" der entsprechenden Anatomie begründet sein.

Beispielsweise ist bekannt, dass eine anatomische Landmarke bzw. ein Oberflächennetz, welche die Lunge betrifft zeitlich instabil ist, während eine anatomische Landmarke bzw. ein Oberflächennetz, welche das Sternum betrifft, zeitlich stabil ist. Die zeitliche Instabilität der Lunge ist in dem anatomisch begründeten Verhalten der Funktion eines "Blasebalgs" begründet. Das anatomisch begründete Verhalten des Sternums, welches eine Stabilisierung des Brustkorbs bewirkt, begründet die zeitliche Stabilität des Sternums.

Es können somit Klassen definiert werden, denen verschiedene Anatomien zugeordnet sind. Beispielsweise kann einer Klasse mit der Bezeichnung "stabil" das Skelett, insbesondere die Wirbelkörper und/oder das Sternum etc. zugeordnet sein. Beispielsweise kann einer Klasse mit der Bezeichnung "instabil" die Lunge und/oder das Herz zugeordnet sein. Insbesondere können der Klasse "instabil" wenigstens eine anatomische Landmarke und/oder wenigstens ein Oberflächennetz zugeordnet sein, die beispielsweise die Lunge und/oder das Herz betrifft. Beispielsweise kann einer Klasse mit der Bezeichnung "relativ stabil" der Darm bzw. wenigstens eine anatomische Landmarke und/oder wenigstens ein Oberflächennetz, die den Darm betrifft, zugeordnet sein.

Basierend auf diesen vordefinierten Klassen kann in dem Verfahrensschritt des Klassifizierens die wenigstens eine anatomische Landmarke und/oder das wenigstens eine Oberflächennetz klassifiziert werden.

Der Einfluss der wenigstens einen anatomischen Landmarke bzw. des wenigstens einen Oberflächennetzes auf das Abweichungsmaß hängt von der entsprechenden zeitlichen Stabilität und damit von der Klasse der wenigstens einen anatomischen Landmarke bzw. des wenigstens einen Oberflächennetzes ab.

Die Erfinder haben erkannt, dass zum Bestimmen bzw. Klassifizieren der zeitlichen Stabilität allgemeingültige Annahmen vorausgesetzt werden können. Die Erfinder haben erkannt, dass es nicht notwendig ist, die zeitliche Stabilität einer anatomischen Landmarke und/oder eines Oberflächennetzes für jeden Patienten erneut in einer longitudinalen bzw. zeitlichen Analyse von Bilddaten zu bestimmen. Die Erfinder haben erkannt, dass auf diese Weise das Verfahren beschleunigt werden kann. Die Erfinder haben außerdem erkannt, dass durch das Klassifizieren basierend auf dem Fachwissen bzw. einer allgemeingültigen Annahme, eine gute Vergleichbarkeit erreicht wird.

Nach einem weiteren Aspekt der Erfindung umfasst der Verfahrensschritt des Zuordnens des medizinischen Bilddatensatzes wenigstens einen der folgenden Schritte:
- Erstellen einer Verknüpfung zwischen dem medizinischen Bilddatensatz und dem medizinischen Vergleichsbilddatensatz in einer Datenbank;
- Bestimmen einer gemeinsamen Identifikationsnummer für den medizinischen Bilddatensatz und den medizinischen Vergleichsbilddatensatz;
- Anwenden einer Funktion zur Bildanalyse auf den medizinischen Vergleichsbilddatensatz und den medizinischen Bilddatensatz.

Dabei wird wenigstens einer der genannten Schritte durchgeführt, wenn das Abweichungsmaß den Schwellenwert unterschreitet. Insbesondere kann dann davon ausgegangen werden, dass der medizinische Bilddatensatz und der medizinische Vergleichsbilddatensatz denselben, insbesondere den ersten, Patienten abbilden.

In dem Verfahrensschritt des Erstellens einer Verknüpfung kann dem medizinischen Bilddatensatz und/oder dem medizinischen Vergleichsbilddatensatz ein Link bzw. ein Verweis zu einem Speicherort des jeweils anderen Bilddatensatz hinzugefügt werden. Mit anderen Worten kann dem medizinischen Bilddatensatz ein Link zu dem Speicherort des medizinischen Vergleichsbilddatensatzes hinzugefügt werden. Alternativ oder zusätzlich kann dem medizinischen Vergleichsbilddatensatz ein Link zu dem Speicherort des medizinischen Bilddatensatzes hinzugefügt werden. Der Speicherort kann dabei als ein Ort in der Datenbank definiert sein. Der Ort kann dabei über den Link adressierbar sein. Mit anderen Worten kann die Verknüpfung ein Verweis auf den jeweils anderen Bilddatensatz sein.

Die Datenbank kann dabei wie oben beschrieben ausgebildet sein. Insbesondere kann die Datenbank zum Speichern von medizinischen Bilddatensätzen ausgebildet sein.

In dem Verfahrensschritt des Bestimmens einer gemeinsamen Identifikationsnummer (Akronym: ID) kann für den medizinischen Bilddatensatz und den medizinischen Vergleichsbilddatensatz eine gemeinsame ID bestimmt werden. Die ID kann dabei eine Rolle einer Patienten-Identifikationsnummer einnehmen. Die ID ist dabei unabhängig von den Patientendaten und wird nur in Abhängigkeit des Abweichungsmaßes im Vergleich zu dem Schwellenwert bestimmt. Die ID kann dabei eine generische Ziffern- und/oder Zahlenfolge sein. Die ID ist dabei insbesondere eindeutig. Mit anderen Worten wird eine ID einmalig an ein Paar bzw. eine Gruppe von einander zugeordneten Bilddatensätzen vergeben.

Beispielsweise durch eine Datenbankabfrage nach der gemeinsamen ID können automatisch alle einander zugeordneten Bilddatensätze in der Datenbank bereitgestellt werden. Insbesondere wird dann der medizinische Bilddatensatz und der medizinische Vergleichsbilddatensatz bereitgestellt.

In dem Verfahrensschritt des Anwendens einer Funktion zur Bildanalyse wird die Funktion zur Bildanalyse auf den medizinischen Bilddatensatz und den medizinischen Vergleichsbilddatensatz angewendet. Dabei wird die Funktion zur Bildanalyse dann angewendet, wenn das Abweichungsmaß den Schwellenwert unterschreitet. Mit anderen Worten wird die Funktion zur Bildanalyse dann angewendet, wenn davon ausgegangen werden kann, dass der medizinische Bilddatensatz und der medizinische Vergleichsbilddatensatz denselben Patienten, insbesondere den ersten Patienten, abbilden. Durch Anwenden der Funktion zur Bildanalyse können beispielsweise der medizinischen Bilddatensatz und der medizinische Vergleichsbilddatensatz verglichen werden. Alternativ oder zusätzlich können durch Anwenden der Funktion zur Bildanalyse der medizinische Bilddatensatz und der medizinische Vergleichsbilddatensatz registriert werden.

Die Erfinder haben erkannt, dass in Abhängigkeit davon, ob der medizinische Bilddatensatz und der medizinische Vergleichsbilddatensatz denselben Patienten abbilden, verschiedene weitere Verfahrensschritte ausgeführt werden können. Die Erfinder haben erkannt, dass durch die Verknüpfung und/oder die gemeinsame ID eine Datenverarbeitung der Bilddatensätze vereinfacht werden kann. Insbesondere ist es auf diese Weise möglich, Daten in der Datenbank zu strukturieren und einen Zugriff zu erleichtern. Beispielsweise kann ein Nutzer, beispielsweise ein Mediziner oder eine medizinische Assistenz, alle Bilder eines Patienten auf einmal aufrufen, wenn die Bilder miteinander über die Verknüpfung und/oder die gemeinsame ID verknüpft sind. Dafür muss der Nutzer keine Patientendaten bereitstellen, es genügt, wenn der Nutzer einen Bilddatensatz des Patienten auswählt. Die anderen Bilddatensätze des Patienten können dann über die Verknüpfung bzw. die Verknüpfungen und/oder die ID abgerufen werden. Die Erfinder haben erkannt, dass eine Bildanalyse durch Anwenden einer Funktion zur Bildanalyse nur dann sinnvoll ist, wenn davon ausgegangen werden kann, dass der medizinische Bilddatensatz und der medizinische Vergleichsbilddatensatz denselben Patienten abbilden.

Nach einem weiteren Aspekt der Erfindung wurde der medizinische Vergleichsbilddatensatz zeitlich vor dem medizinischen Bilddatensatz erfasst. Dabei wird durch das Anwenden der Funktion zur Bildanalyse auf den medizinischen Vergleichsbilddatensatz und den medizinischen Bilddatensatz eine zeitliche Veränderung zwischen dem medizinischen Vergleichsbilddatensatz und dem medizinischen Bilddatensatz bestimmt.

Der medizinische Bilddatensatz und der medizinische Vergleichsbilddatensatz bilden dabei insbesondere denselben Patienten, insbesondere den ersten Patienten, ab. Dabei ist das Abweichungsmaß insbesondere kleiner als der Schwellenwert. Der medizinische Bilddatensatz kann beispielsweise eine Woche, einen Monat, ein viertel Jahr, ein halbes Jahr, ein Jahr etc. nach dem medizinischen Vergleichsbilddatensatz erfasst worden sein. Insbesondere kann der medizinische Bilddatensatz im Zuge einer Folge-Untersuchung bzw. Follow-Up Untersuchung des ersten Patienten erfasst worden sein. Insbesondere kann dann die Funktion zur Bildanalyse für eine Follow-Up Analyse ausgebildet sein.

Insbesondere kann durch Anwenden der Funktion zur Bildanalyse für die Follow-Up Analyse der medizinische Vergleichsbilddatensatz mit dem medizinischen Bilddatensatz verglichen werden.

Insbesondere kann durch das Anwenden der Funktion zur Bildanalyse im Zuge der Follow-Up Analyse beispielsweise eine Veränderung einer Läsion bestimmt werden. Dabei kann die Läsion in dem medizinischen Vergleichsbilddatensatz und/oder in dem medizinischen Bilddatensatz abgebildet sein. Die Läsion kann beispielsweise ein Tumor, ein Aneurysma und/oder eine Veränderung einer anatomischen Struktur wie bei einer Osteoporose oder bei COPD (Akronym für Chronical Obstructive Pulmonary Disease) etc. sein. Die Funktion zur Bildanalyse kann beispielsweise dazu ausgebildet sein, eine Größe bzw. ein Volumen der Läsion in dem medizinischen Vergleichsbilddatensatz und dem medizinischen Bilddatensatz zu bestimmen und/oder zu vergleichen.

Alternativ oder zusätzlich kann die Funktion zur Bildanalyse dazu ausgebildet sein Läsionen zu bestimmen, die nur in dem medizinischen Bilddatensatz oder nur in dem medizinischen Vergleichsbilddatensatz abgebildet sind. Insbesondere können auf diese Weise beispielsweise neu entstandene Metastasen bestimmt werden. Insbesondere können beispielsweise Metastasen bestimmt werden, die beispielsweise unter einer Chemotherapie und/oder eine Strahlentherapie verschwunden sind.

Die Funktion zur Bildanalyse kann somit für eine longitudinale Analyse bzw. eine zeitliche Analyse von Bilddatensätzen ausgebildet sein.

Die Erfinder haben erkannt, dass mittels des Verfahrens Bilddatensätze bestimmt werden können, die für eine Follow-Up Analyse durch die Funktion zur Bildanalyse geeignet sind. Dabei sind insbesondere Bilddatensätze geeignet, die denselben Patienten abbilden. Diese Bilddatensätze können durch Ausführen des oben beschriebenen Verfahrens ermittelt bzw. bestimmt werden.

Nach einem weiteren Aspekt der Erfindung ist der medizinische Bilddatensatz dem medizinischen Vergleichsbilddatensatz durch eine vorläufige Zuordnung zugeordnet. Dabei umfasst das Verfahren weiterhin einen Verfahrensschritt eines Korrigierens der vorläufigen Zuordnung zwischen dem medizinischen Bilddatensatz und dem medizinischen Vergleichsbilddatensatz, wenn das Abweichungsmaß größer oder gleich dem Schwellenwert ist.

Die vorläufige Zuordnung erlaubt ein automatisches Auffinden des medizinischen Bilddatensatzes ausgehende von dem medizinischen Vergleichsbilddatensatz. Alternativ oder zusätzlich erlaubt die vorläufige Zuordnung ein automatisches Auffinden des medizinischen Vergleichsbilddatensatzes ausgehende von dem medizinischen Bilddatensatz.

Die vorläufige Zuordnung kann insbesondere eine vorläufige Verknüpfung des medizinischen Bilddatensatzes und des medizinischen Vergleichsbilddatensatzes sein. Die vorläufige Verknüpfung kann dabei wie oben bezüglich der Verknüpfung beschrieben ausgebildet sein. Die vorläufige Verknüpfung kann insbesondere auf einen Speicherort des medizinischen Bilddatensatzes und/oder des medizinischen Vergleichsbilddatensatzes verweisen. Die vorläufige Verknüpfung kann dabei von dem medizinischen Bilddatensatz und/oder von dem medizinischen Vergleichsbilddatensatz umfasst sein. Die vorläufige Verknüpfung kann dabei einen Link zu dem jeweils anderen Bilddatensatz umfassen. Mit anderen Worten kann die von dem medizinischen Bilddatensatz umfasste vorläufige Verknüpfung einen Link zu dem medizinischen Vergleichsbilddatensatz umfassen. Alternativ oder zusätzlich kann die von dem medizinischen Vergleichsbilddatensatz umfasste vorläufige Verknüpfung einen Link zu dem medizinischen Bilddatensatz umfassen.

Alternativ oder zusätzlich kann die vorläufige Zuordnung durch eine vorläufige gemeinsame Identifikationsnummer realisiert sein. Die vorläufige Identifikationsnummer kann dabei wie oben in Bezug auf die Identifikationsnummer beschrieben ausgebildet sein. Die vorläufige Identifikationsnummer bzw. vorläufige ID kann dabei insbesondere eindeutig sein. Die vorläufige ID kann dabei für den vorläufig einander zugeordneten medizinischen Bilddatensatz und den medizinischen Vergleichsbilddatensatz identisch sein. Insbesondere können somit durch einer Abfrage nach allen Bilddatensätzen mit der gleichen vorläufigen ID, alle durch eine vorläufige Zuordnung einander zugeordneten Bilddatensätze bestimmt werden. Dabei umfasst der medizinische Bilddatensatz die vorläufige ID. Dabei umfasst der medizinische Vergleichsbilddatensatz die vorläufige ID. Alle Bilddatensätze, die dieselbe vorläufige ID umfassen, sind einander zugeordnet.

Der Verfahrensschritt des Korrigierens der vorläufigen Zuordnung zwischen dem medizinischen Bilddatensatz und dem medizinischen Vergleichsbilddatensatz wird dann ausgeführt, wenn das Abweichungsmaß größer oder gleich dem Schwellenwert ist.

Insbesondere kann davon ausgegangen werden, dass der medizinische Bilddatensatz und der medizinische Vergleichsbilddatensatz unterschiedliche Patienten betreffen, wenn das Abweichungsmaß größer oder gleich dem Schwellenwert ist. Insbesondere kann dann der medizinische Vergleichsbilddatensatz wenigstens einen Körperteil des ersten Patienten abbilden und der medizinische Bilddatensatz kann wenigstens einen Körperteil des zweiten Patienten abbilden.

Alternativ kann davon ausgegangen werden, dass sich der abgebildete Patient stark zwischen dem Erfassen des medizinischen Vergleichsbilddatensatz und dem medizinischen Bilddatensatz verändert hat, wenn beide Bilddatensätze wenigstens einen Körperteil des ersten Patienten abbilden und das Abweichungsmaß größer oder gleich dem Schwellenwert ist.

Das Korrigieren der vorläufigen Zuordnung kann dabei insbesondere ein Löschen der vorläufigen Zuordnung umfassen. Insbesondere kann das ein Löschen der vorläufigen Verknüpfung und/oder der vorläufigen ID umfassen.

Alternativ oder zusätzlich kann das Korrigieren der vorläufigen Zuordnung das Bereitstellen einer Korrekturinformation für einen Nutzer sein. Die Korrekturinformation kann dabei angeben, dass die vorläufige Zuordnung als falsch identifiziert wurde. In Ausführungen der Erfindung kann der Nutzer diese Korrekturinformation bestätigen oder verwerfen. Wird eine Bestätigungsinformation empfangen, kann die vorläufige Zuordnung gelöscht werden. Wird eine Verwerfungsinformation empfangen, kann die vorläufige Zuordnung bestehen bleiben, auch wenn das Abweichungsmaß größer oder gleich dem Schwellenwert ist. Der Nutzer kann dabei beispielsweise ein Mediziner, eine medizinische Assistenz etc. sein.

Die Erfinder haben erkannt, dass durch das Korrigieren der vorläufigen Zuordnung eine falsche Zuordnung korrigiert werden kann. Mit dem beschriebenen Verfahren kann somit überprüft werden, ob der medizinische Bilddatensatz und der medizinische Vergleichsbilddatensatz tatsächlich denselben Patienten betreffen. Die Erfinder haben erkannt, dass bei einem Abweichungsmaß größer oder gleich dem Schwellenwert nicht davon ausgegangen werden kann, dass der medizinische Bilddatensatz und der medizinische Vergleichsbilddatensatz denselben Patienten betreffen. Um zu verhindern, dass beispielsweise eine Follow-Up Bildanalyse basierend auf Bilddatensätzen, die unterschiedliche Patienten betreffen ausgeführt werden, wird die vorläufige Zuordnung korrigiert. Auch wenn das Abweichungsmaß groß ist, obwohl der medizinische Bilddatensatz und der medizinische Vergleichsbilddatensatz denselben Patienten betreffen, weil der Patient sich stark verändert hat, kann die vorläufige Zuordnung wie oben beschrieben korrigiert werden. Die Erfinder haben erkannt, dass in diesem Fall automatisierte Follow-Up Bildanalysen möglicherweise falsche Ergebnisse ausgeben, wenn die Veränderung des Patienten zu groß ist.

Nach einem weiteren Aspekt der Erfindung umfasst das Verfahren weiterhin einen Verfahrensschritt eines Bereitstellens eines Warnsignals, wenn das Abweichungsmaß größer oder gleich dem Schwellenwert ist.

Das Warnsignal kann insbesondere ein akustisches und/oder ein optisches Warnsignal sein. Insbesondere kann der Nutzer mit dem Warnsignal darüber informiert werden, dass das Abweichungsmaß größer oder gleich dem Schwellenwert ist.

Alternativ oder zusätzlich kann das Warnsignal die oben beschriebene Korrekturinformation umfassen. Das Bereitstellen des Warnsignals kann somit ein Bereitstellend er Korrekturinformation umfassen.

Das Warnsignal kann dabei insbesondere mittels einer Anzeigeeinheit bereitgestellt werden. Mit anderen Worten kann das Bereitstellen des Warnsignals ein Anzeigen des Warnsignals umfassen. Die Anzeigeeinheit kann dabei insbesondere ein Bildschirm bzw. ein Monitor sein. Alternativ oder zusätzlich kann das Warnsignal mittels eines Lautsprechers bereitgestellt werden. Dabei kann das Warnsignal mittels Sprachausgabe bereitgestellt werden. Alternativ oder zusätzlich kann das Warnsignal einen Warnton umfassen, der mit dem Lautsprecher ausgegeben wird. Alternativ oder zusätzlich kann das Warnsignal mit einer Leuchte bereitgestellt werden. Beispielsweise kann die Leuchte rot leuchten oder blinken, wenn das Abweichungsmaß größer oder gleich dem Schwellenwert ist.

Die Erfinder haben erkannt, dass der Nutzer mittels des Warnsignals gewarnt werden kann, dass eine Follow-Up Bildanalyse möglicherweise zu falschen Ergebnissen führt, wenn das Abweichungsmaß den Schwellenwert überschreitet. Außerdem haben die Erfinder erkannt, dass mittels des Warnsignals der Nutzer darauf aufmerksam gemacht werden kann, eine vorläufige Zuordnung zu überprüfen. Die Erfinder haben erkannt, dass auf diese Weise nahezu ausgeschlossen werden kann, dass Bilddatensätze von verschiedenen Patienten fälschlicherweise einander zugeordnet bzw. miteinander verknüpft werden.

Nach einem optionalen Aspekt des Verfahrens umfassen der medizinische Bilddatensatz und der medizinische Vergleichsbilddatensatz dreidimensionale medizinische Bilder.

Insbesondere ist die von dem medizinischen Bilddatensatz umfasste Abbildung ein dreidimensionales medizinisches Bild. Insbesondere ist die von dem medizinischen Vergleichsbilddatensatz umfasste Abbildung ein dreidimensionales medizinisches Bild.

Das dreidimensionale medizinische Bild bildet dabei insbesondere den wenigstens einen Körperteil des ersten bzw. zweiten Patienten in drei Raumrichtungen ab. Dabei umfasst das dreidimensionale medizinische Bild eine Mehrzahl an Voxeln, die in einer Voxelmatrix angeordnet sind. Jeder Voxel umfasst dabei einen Voxelwert.

Das dreidimensionale medizinische Bild kann insbesondere ein Computer-Tomographie Bild oder ein Magnet-Resonanz-Tomographie Bild oder ein Tomosynthese Bild oder ein Positronen-Emissions-Tomographie Bild oder ein Einzel-Photonen-Emissions-Computer-Tomographie Bild etc. sein. Mit anderen Worten kann das medizinische Bild insbesondere mit einem Computer-Tomographie-System oder einem Magnet-Resonanz-Tomographie-System oder einem Mammographie-System oder einem Positronen-Emissions-Tomographie-System oder einem Einzel-Photonen-Emissions-Computer-Tomographie-System oder einem C-Bogen-System erfasst worden sein.

Die Erfinder haben erkannt, dass die biometrischen Daten bzw. die biometrischen Vergleichsdaten vorteilhafterweise aus dreidimensionalen medizinischen Bilddaten extrahiert werden. Die Erfinder haben erkannt, dass die biometrischen Daten bzw. die biometrischen Vergleichsdaten dann am wenigstens fehlerbehaftet sind. Die Erfinder haben erkannt, dass eine zweidimensionale Projektion der biometrischen Daten bzw. der biometrischen Vergleichsdaten zu einer Verzerrung führen kann.

Die Erfindung betrifft optional ein computerimplementiertes Verfahren zum Zuordnen eines medizinischen Bilddatensatzes zu einem Vergleichsbilddatensatz in Abhängigkeit von biometrischen Daten. Das Verfahren umfasst einen Verfahrensschritt eines Empfangens des medizinischen Bilddatensatzes. Das Verfahren umfasst weiterhin einen Verfahrensschritt eines Empfangens mehrerer möglicher Vergleichsbilddatensätze. Das Verfahren umfasst weiterhin ein Extrahieren von biometrischen Daten basierend auf dem medizinischen Bilddatensatz. Das Verfahren umfasst weiterhin ein Extrahieren von möglichen biometrischen Vergleichsdaten basierend auf jedem der möglichen Vergleichsbilddatensätze. Das Verfahren umfasst weiterhin einen Verfahrensschritt eines Bestimmens von Abweichungsmaßen zwischen den biometrischen Daten und jedem der möglichen biometrischen Vergleichsdaten. Das Verfahren umfasst weiterhin einen Verfahrensschritt eines Auswählens eines oder mehrerer medizinischer Vergleichsbilddatensätze aus den möglichen medizinischen Vergleichsbilddatensätzen basierend auf den Abweichungsmaßen. Das Verfahren umfasst weiterhin einen Verfahrensschritt eines Zuordnens des medizinischen Bilddatensatzes zu dem einen oder mehreren ausgewählten medizinischen Vergleichsbilddatensätzen.

Die obige Beschreibung kann auf diese Ausführung der Erfindung analog übertragen werden. Die Ausführung der Erfindung kann mit allen oben beschriebenen Aspekten kombiniert werden.

In dem Verfahrensschritt des Auswählens können insbesondere diejenigen medizinischen Vergleichsbilddatensätze ausgewählt werden, deren Abweichungsmaße den oben beschriebenen Schwellenwert unterschreiten.

Die Erfinder haben erkannt, dass das Verfahren genutzt werden kann, um einen medizinischen Bilddatensatz den passenden medizinischen Vergleichsbilddatensätzen aus einer größeren Menge an Vergleichsbilddatensätzen zuzuordnen. Die Erfinder haben erkannt, dass das Verfahren somit auch angewendet werden kann, wenn bereits mehr als ein medizinischer Vergleichsbilddatensatz eines Patienten erfasst wurde.

Die Erfindung betrifft außerdem ein Zuordnungssystem zum Zuordnen eines medizinischen Bilddatensatzes zu einem medizinischen Vergleichsbilddatensatz in Abhängigkeit von biometrischen Daten. Das Zuordnungssystem umfasst eine Schnittstelle und eine Recheneinheit. Die Schnittstelle ist zum Empfangen des medizinischen Bilddatensatzes ausgebildet. Die Schnittstelle ist weiterhin zum Empfangen wenigstens eines medizinischen Vergleichsbilddatensatzes ausgebildet. Die Recheneinheit ist zum Extrahieren von biometrischen Daten basierend auf dem medizinischen Bilddatensatz ausgebildet. Die Recheneinheit ist weiterhin zum Extrahieren von biometrischen Vergleichsdaten basierend auf dem medizinischen Vergleichsbilddatensatz ausgebildet. Die Recheneinheit ist weiterhin zum Bestimmen eines Abweichungsmaßes zwischen den biometrischen Vergleichsdaten und den biometrischen Daten ausgebildet. Die Recheneinheit ist weiterhin zum Zuordnen des medizinischen Bilddatensatzes zu dem medizinischen Vergleichsbilddatensatz ausgebildet, wenn das Abweichungsmaß einen Schwellenwert unterschreitet.

Ein solches Zuordnungssystem kann insbesondere dazu ausgebildet sein, das zuvor beschriebene Verfahren zum Zuordnen eines medizinischen Bilddatensatzes zu einem medizinischen Vergleichsbilddatensatz in Abhängigkeit von biometrischen Daten und seine Aspekte auszuführen. Das Zuordnungssystem ist dazu ausgebildet, dieses Verfahren und seine Aspekte auszuführen, indem die Schnittstelle und die Recheneinheit ausgebildet sind, die entsprechenden Verfahrensschritte auszuführen.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendeten Zuordnungssysteme auf einfache Weise durch ein Software-Update nachgerüstet werden können, um auf die beschriebene Weise zu arbeiten. Ein solches Computerprogrammprodukt kann neben dem Computerprogramm gegebenenfalls zusätzliche Bestandteile wie z. **B.** eine Dokumentation und/oder zusätzliche Komponenten, sowie Hardware-Komponenten, wie z.B. Hardware-Schlüssel (Dongles etc.) zur Nutzung der Software, umfassen.

Insbesondere betrifft die Erfindung auch ein Computerprogrammprodukt mit einem Computerprogramm, welches direkt in einen Speicher eines Zuordnungssystems ladbar ist, mit Programmabschnitten, um alle Schritte des oben beschriebenen Verfahrens zum Zuordnen eines medizinischen Bilddatensatzes zu einem medizinischen Vergleichsbilddatensatz in Abhängigkeit von biometrischen Daten und seine Aspekte auszuführen, wenn die Programmabschnitte von dem Zuordnungssystem ausgeführt werden.

Insbesondere betrifft die Erfindung ein computerlesbares Speichermedium, auf welchem von einem Zuordnungssystem lesbare und ausführbare Programmabschnitte gespeichert sind, um alle Schritte des oben beschriebenen Verfahrens zum Zuordnen eines medizinischen Bilddatensatzes zu einem medizinischen Vergleichsbilddatensatz in Abhängigkeit von biometrischen Daten und seine Aspekte auszuführen, wenn die Programmabschnitte von dem Zuordnungssystem ausgeführt werden.

Die oben beschriebenen Eigenschaften, Merkmale und Vorteile dieser Erfindung werden klarer und verständlicher im Zusammenhang mit folgenden Figuren und ihren Beschreibungen. Dabei sollen die Figuren und Beschreibungen die Erfindung und ihre Ausführungsformen in keiner Weise einschränken.

In verschiedenen Figuren sind gleiche Komponenten mit korrespondierenden Bezugszeichen versehen. Die Figuren sind in der Regel nicht maßstabsgetreu.

Es zeigen
- Fig. 1: ein erstes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten,
- Fig. 2: ein zweites Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten,
- Fig. 3: ein drittes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten,
- Fig. 4: ein viertes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten,
- Fig. 5: ein fünftes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten,
- Fig. 6: ein sechstes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten,
- Fig. 7: ein Zuordnungssystem zum Zuordnen eines medizinischen Bilddatensatzes zu einem medizinischen Vergleichsbilddatensatz in Abhängigkeit von biometrischen Daten.

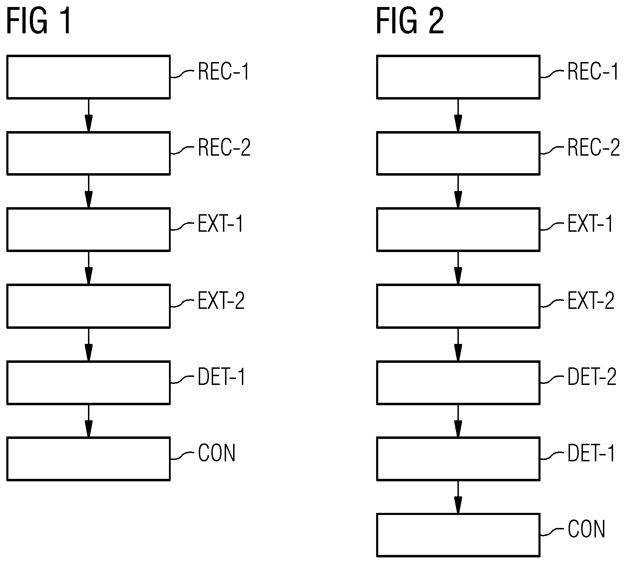

**Figur 1** zeigt ein erstes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten.

In einem Verfahrensschritt eines Empfangens REC-1 eines medizinischen Bilddatensatzes wird der medizinische Bilddatensatz mittels einer Schnittstelle SYS.IF empfangen. Der medizinische Bilddatensatz umfasst dabei eine Abbildung bzw. ein medizinisches Bild eines ersten oder eines zweiten Patienten. Das medizinische Bild bildet dabei wenigstens einen Teil, insbesondere wenigstens einen Körperteil des ersten oder zweiten Patienten ab. Mit anderen Worten bildet der medizinische Bilddatensatz den ersten oder zweiten Patienten ab. Der Körperteil kann dabei beispielsweise ein Thorax, ein Abdomen, ein Kopf, ein Arm oder ein Bein des ersten oder zweiten Patienten sein.

Das medizinische Bild wurde mit einem medizinischen Bildgebungssystem erfasst. Das medizinische Bildgebungssystem kann insbesondere ein Röntgen-System, ein Mammographie-System, ein Computer-Tomographie-System, ein Magnet-Resonanz-Tomographie-System, ein C-Bogen-System, ein Ultraschall-System, ein Positronen-Emissions-Tomographie-System oder ein Einzel-Photonen-Emissions-Computer-Tomographie-System etc. sein. Das medizinische Bild ist ein dreidimensionales medizinisches Bild. In alternativen Ausführungen der Erfindung kann das medizinische Bild ein zweidimensionales medizinisches Bild sein. Das dreidimensionale medizinische Bild umfasst eine Mehrzahl von Voxeln, die in einer dreidimensionalen Voxelmatrix angeordnet sind. Jeder Voxel umfasst einen Voxelwert. Der Voxelwert ist ein Messwert, beispielsweise ein Intensitätswert, der eine Eigenschaft des in dem Voxel abgebildeten Materials darstellt. Wenn das medizinische Bild zweidimensional ist, umfasst das medizinische Bild eine zweidimensionale Pixelmatrix mit einer Mehrzahl von Pixeln. Jedem Pixel ist dabei ein Pixelwert zugeordnet. Das medizinische Bild ist insbesondere in einem DICOM Format.

Der medizinische Bilddatensatz ist insbesondere anonymisiert oder pseudonymisiert. Mit anderen Worten sind Patientendaten in dem medizinischen Bilddatensatz entweder entfernt oder durch ein Pseudonym ersetzt. Alternativ können die Patientendaten verschlüsselt sein. Es ist somit nicht möglich, basierend auf dem medizinischen Bilddatensatz auf eine Identität des ersten oder zweiten Patienten zurückzuschließen. Die Patientendaten sind dabei typischerweise von einem DICOM Header umfasst.

Der medizinische Bilddatensatz wird von einer Datenbank bereitgestellt. Mit anderen Worten wird der medizinische Bilddatensatz mit der Schnittstelle SYS.IF von der Datenbank empfangen. Alternativ kann der medizinische Bilddatensatz von dem medizinischen Bildgebungssystem bereitgestellt werden. Alternativ kann der medizinische Bilddatensatz von einem Nutzer bereitgestellt werden. Der Nutzer ist dabei insbesondere ein Mediziner oder eine medizinische Assistenz. Alternativ kann der Nutzer der erste oder zweite Patient sein.

Die Datenbank ist ein Bildarchivierungs-und-KommunikationsSystem (engl. Picture Archiving and Communications System, Akronym: PACS). Alternativ kann die Datenbank beispielsweise ein Krankenhausinformationssystem (Akronym: KIS) oder ein Radiologieinformationssystem (Akronym: RIS) sein.

Die Datenbank kann auf einem lokalen Server betrieben (engl: hosted) werden. Alternativ kann die Datenbank in einem Cloudsystem betrieben werden.

In einem Verfahrensschritt eines Empfangens REC-2 des medizinischen Vergleichsbilddatensatzes wird der medizinische Vergleichsbilddatensatz mit der Schnittstelle SYS.IF empfangen.

Der medizinische Vergleichsbilddatensatz ist analog zu dem medizinischen Bilddatensatz ausgebildet. Der medizinische Vergleichsbilddatensatz umfasst ebenfalls ein medizinisches Bild, welches mit einem medizinischen Bildgebungssystem erfasst wurde. Das medizinische Bildgebungssystem kann dabei gleich oder verschieden von dem medizinischen Bildgebungssystem sein, mit dem das medizinische Bild des medizinischen Bilddatensatzes erfasst wurde.

Das medizinische Bild des medizinischen Vergleichsbilddatensatzes bildet den ersten Patienten ab. Mit anderen Worten bildet der medizinische Vergleichsbilddatensatz den ersten Patienten ab. Dabei ist wenigstens ein Teil insbesondere ein Körperteil des ersten Patienten abgebildet. Der Körperteil kann gleich oder verschieden von dem Körperteil sein, den der medizinische Bilddatensatz abbildet.

Der medizinische Vergleichsbilddatensatz wird dabei von der Datenbank oder dem medizinischen Bildgebungssystem oder dem Nutzer bereitgestellt.

In einem Verfahrensschritt eines Extrahierens EXT-1 von biometrischen Daten werden die biometrischen Daten basierend auf dem medizinischen Bilddatensatz extrahiert.

Dabei können die biometrischen Daten bereits von dem medizinischen Bilddatensatz umfasst sein. Dann umfasst das Extrahieren EXT-1 der biometrischen Daten ein Auslesen der biometrischen Daten aus dem medizinischen Bilddatensatz.

Alternativ können die biometrischen Daten basierend auf dem medizinischen Bilddatensatz bestimmt werden. Dann umfasst das Extrahieren EXT-1 der biometrischen Daten ein Bestimmen der biometrischen Daten.

Die biometrischen Daten beschreiben eine Eigenschaft des in dem medizinischen Bilddatensatz abgebildeten ersten oder zweiten Patienten. Die Eigenschaft ist dabei insbesondere einzigartig bzw. kennzeichnend für den Patienten. Die biometrischen Daten ähneln dabei einem Fingerabdruck. Die biometrischen Daten beschreiben dabei beispielsweise eine anatomische Eigenschaft oder Besonderheit des ersten oder zweiten Patienten. Die biometrischen Daten können beispielsweise angeben, wenn der erste oder zweite Patient nur eine Niere besitzt. Alternativ oder zusätzlich können die biometrischen Daten körperliche Eigenschaften wie eine Größe des ersten oder zweiten Patienten angeben. Alternativ oder zusätzlich können die biometrischen Daten eine Anordnung von Anatomien beispielsweise der Organe und/oder des Skeletts des ersten oder zweiten Patienten angeben.

In Ausführungen der Erfindung umfassen die biometrischen Daten wenigstens eine anatomische Landmarke und/oder wenigstens ein Oberflächennetz wenigstens einer Anatomie des abgebildeten Körperteils in dem medizinischen Bilddatensatz.

Die anatomische Landmarke gibt dabei eine Position einer Anatomie in dem medizinischen Bild des medizinischen Bilddatensatzes an. Die Position kann dabei in Ausführungen der Erfindung durch eine Koordinate der anatomischen Landmarke in dem medizinischen Bild angegeben werden. Die Koordinate ist bei einem dreidimensionalen medizinischen Bild ein Tripel. Mit anderen Worten umfasst die Koordinate bei einem dreidimensionalen medizinischen Bild ein Tripel von Werten, bei denen jeder Wert die Position der anatomischen Landmarke in dem medizinischen Bild in eine Raumrichtung angibt. Wenn das medizinische Bild zweidimensional ist, umfasst die Koordinate ein Wertepaar. Die anatomische Landmarke betrifft somit eine Anatomie. Die Anatomie kann dabei beispielsweise eine der folgenden Anatomien sein: Zentrum des Aortenbogens, Aortenwurzel, Verzweigung der Arteria brachiocephalica, Verzweigung der linken Arteria Subclavia und der Arteria Vertebralis, Verzweigung der rechten Arteria Subclavia und der Arteria Vertebralis, Truncus coeliacus, linke Halsschlagader, Carina Tracheae, Herz, obere Spitze der linken Niere, obere Spitze der rechten Niere, obere Spitze des linken Lungenflügels, obere Spitze des rechten Lungenflügels, Zentrum der Leber, obere Spitze der Leber, Bauchspeicheldrüse, Abgang der Nierenarterie, Spitze des Sternum, Wirbelkörper: C7, T1, T2, T3, T4, T5, T6, T7, T8, T9, T10, T11, T12, L1. Die biometrischen Daten können insbesondere mehr als eine anatomische Landmarke umfassen. Dann betreffen jeweils zwei anatomische Landmarken zwei unterschiedliche Anatomien.

Die wenigstens eine anatomische Landmarke in dem medizinischen Vergleichsbilddatensatz bzw. in dem medizinischen Bilddatensatz kann beispielsweise wie in "Ghesu FC, Georgescu B, Zheng Y, Grbic S, Maier A, Hornegger J, Comaniciu D, "Multi-Scale Deep Reinforcement Learning for Real-Time 3D-Landmark Detection in CT Scans", IEEE Trans Pattern Anal Mach Intell. 2017" beschrieben, bestimmt worden sein.

Alternativ oder zusätzlich umfassen die biometrischen Daten wenigstens ein Oberflächennetz. Das Oberflächennetz beschreibt dabei die Oberfläche einer in dem medizinischen Bild abgebildeten Anatomie des ersten oder zweiten Patienten. Das Oberflächennetz betrifft somit eine Anatomie des ersten oder zweiten Patienten. Das Oberflächennetz umfasst dabei mehr als einen Punkt auf der Oberfläche der Anatomie. Das Oberflächennetz kann in Ausführungen der Erfindung die Punkte in Form von Koordinaten in dem medizinischen Bild umfassen. Das Oberflächennetz umfasst dabei mehr als eine Koordinate. Die Koordinate ist dabei wie oben beschrieben ausgebildet. Die Anatomie, die das Oberflächennetz betrifft, kann dabei beispielsweise der linke obere Lungenlappen, der linke untere Lungenlappen, der rechte obere Lungenlappen, der rechte mittlere Lungenlappen, der rechte untere Lungenlappen, das Herz, die Aorta, die Wirbelkörper sein. Das wenigstens eine Oberflächennetz kann basierend auf einer Segmentierung der Anatomie in dem medizinischen Bild bestimmt worden sein. Die biometrischen Daten können mehr als ein Oberflächennetz umfassen. Dabei betreffen verschiedene Oberflächennetze verschiedene Anatomien bzw. verschiedene Bereiche von Anatomien.

Wenn die biometrischen Daten mehr als eine anatomische Landmarke und/oder mehr als ein Oberflächennetz umfassen, beschreiben die biometrischen Daten eine räumliche Beziehung zwischen den anatomischen Landmarken und/oder Oberflächennetzen. Die räumliche Beziehung beschreibt dabei einen Abstand zwischen den einzelnen anatomischen Landmarken und/oder Oberflächennetzen. Dabei kann der Abstand insbesondere in metrischen oder anglo-amerikanischen Einheiten angegeben sein. Die biometrischen Daten umfassen den Abstand bzw. die Abstände wenigstens indirekt, wenn sie die Koordinaten der anatomischen Landmarken und/oder Oberflächennetze umfassen. Die räumliche Beziehung kann außerdem eine räumliche Anordnung der anatomischen Landmarken und/oder Oberflächennetze beschreiben. Die räumliche Anordnung kann dabei insbesondere durch die Koordinaten gegeben sein. Die räumliche Anordnung beschreibt die räumliche Lage der anatomischen Landmarken und/oder Oberflächennetze zueinander im zwei- oder dreidimensionalen Raum. Die räumliche Beziehung kann außerdem die Form der Oberflächennetze berücksichtigen.

In einem Verfahrensschritt eines Extrahierens EXT-2 von biometrischen Vergleichsdaten werden die biometrischen Vergleichsdaten basierend auf dem medizinischen Vergleichsbilddatensatz extrahiert.

Wie oben bezüglich des Extrahierens EXT-1 der biometrischen Daten beschrieben, kann auch das Extrahieren EXT-2 der biometrischen Vergleichsdaten ein Auslesen oder ein Bestimmen der biometrischen Vergleichsdaten aus dem medizinischen Vergleichsbilddatensatz umfassen.

Die biometrischen Vergleichsdaten können dabei in Bezug auf den wenigstens einen in dem medizinischen Vergleichsbilddatensatz abgebildeten Körperteil des ersten Patienten analog zu den biometrischen Daten ausgebildet sein. Insbesondere können in Ausführungen der Erfindung die biometrischen Vergleichsdaten wenigstens eine anatomische Landmarke und/oder wenigstens ein Oberflächennetz umfassen. Dabei kann die wenigstens eine anatomische Landmarke und/oder das wenigstens eine Oberflächennetz in Ausführungen der Erfindung eine Koordinate in dem von dem medizinischen Vergleichsbilddatensatz umfassten medizinischen Bild umfassen. Dabei kann das wenigstens eine Oberflächennetz insbesondere mehr als eine Koordinate umfassen.

Die biometrischen Vergleichsdaten umfassen dabei wenigstens eine anatomische Landmarke und/oder wenigstens ein Oberflächennetz, das dieselbe Anatomie betrifft wie wenigstens eine von den biometrischen Daten umfasste anatomische Landmarke und/oder Oberflächennetz.

In einem Verfahrensschritt eines Bestimmens DET-1 eines Abweichungsmaßes wird das Abweichungsmaß zwischen den biometrischen Vergleichsdaten und den biometrischen Daten bestimmt.

Das Abweichungsmaß gibt dabei an, wie stark die biometrischen Daten von den biometrischen Vergleichsdaten abweichen. Das Abweichungsmaß ist dabei gegenteilig zu einem Ähnlichkeitsmaß. Das Abweichungsmaß gibt die Abweichung beispielsweise als reelle Zahl oder als Prozentzahl oder als Kategorie an. Dabei gilt: je größer das Abweichungsmaß, desto größer die Abweichung, desto geringer die Ähnlichkeit der biometrischen Daten und der biometrischen Vergleichsdaten. Die Kategorie kann dabei eine von mehreren hierarchisch strukturierten Kategorien sein. Dabei gilt: je höher die Kategorie in der Hierarchie, desto größer ist die Abweichung, desto geringer ist die Ähnlichkeit der biometrischen Daten und der biometrischen Vergleichsdaten. Die Kategorien können hierarchisch aufsteigend beispielsweise folgendermaßen lauten: "kaum Abweichungen", "mittel", "große Abweichung".

In einem Verfahrensschritt eines Zuordnens CON des medizinischen Bilddatensatzes zu dem medizinischen Vergleichsbilddatensatz wird der medizinische Bilddatensatz dem medizinischen Vergleichsbilddatensatz zugeordnet, wenn das Abweichungsmaß einen Schwellenwert unterschreitet.

Der Schwellenwert kann dabei ebenfalls als reelle Zahl oder als Prozentzahl oder als Kategorie ausgebildet sein. Dabei ist der Schwellenwert analog zu dem Abweichungsmaß ausgebildet. Der Schwellenwert ist empirisch bestimmt. Alternativ kann der Schwellenwert durch maschinelles Lernen bestimmt sein. Der Schwellenwert gibt an, wie groß das Abweichungsmaß maximal sein darf, damit davon ausgegangen werden kann, dass der medizinische Bilddatensatz ebenso wie der medizinische Vergleichsbilddatensatz den ersten Patienten abbildet. Wenn das Abweichungsmaß den Schwellenwert unterschreitet bzw. kleiner als der Schwellenwert ist, kann davon ausgegangen werden, dass der medizinische Bilddatensatz und der medizinische Vergleichsbilddatensatz denselben Patienten abbilden bzw. ein medizinisches Bild desselben Patienten umfassen. Wenn des Abweichungsmaß größer oder gleich dem Schwellenwert ist, kann davon ausgegangen werden, dass der medizinische Bilddatensatz und der medizinische Vergleichsbilddatensatz verschiedene Patienten abbilden, oder dass sie denselben Patienten abbilden, der sich zwischen dem Erfassen der beiden Bilddatensätze stark verändert hat. Wenn das Abweichungsmaß und der Schwellenwert in Form einer Kategorie ausgebildet sind, unterschreitet das Abweichungsmaß den Schwellenwert, wenn es einer Kategorie mit niedrigerer Hierarchie zugeordnet ist als der Schwellenwert.

**Figur 2** zeigt ein zweites Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten.

Die Verfahrensschritte des Empfangens REC-1 des medizinischen Bilddatensatzes, des Empfangens REC-2 des medizinischen Vergleichsbilddatensatzes, des Extrahierens EXT-1 der biometrischen Daten, des Extrahierens EXT-2 der biometrischen Vergleichsdaten, des Bestimmens DET-1 des Abweichungsmaßes und des Zuordnens CON des medizinischen Bilddatensatzes zu dem medizinischen Vergleichsbilddatensatz sind analog zu der Beschreibung gemäß Figur 1 ausgebildet.

Das Verfahren umfasst in diesem Ausführungsbeispiel außerdem einen Verfahrensschritt eines Herstellens DET-2 einer Registrierung zwischen den biometrischen Vergleichsdaten und den biometrischen Daten. Der Verfahrensschritt des Bestimmens DET-1 des Abweichungsmaßes erfolgt dabei basierend auf der Registrierung.

Beim Herstellen DET-2 der Registrierung werden die biometrischen Daten und die biometrischen Vergleichsdaten in einen räumlichen Zusammenhang bzw. in eine räumliche Beziehung zueinander gebracht. Auf dieser Weise kann eine räumliche Eigenschaft insbesondere eine räumliche Beziehung der biometrischen Daten mit einer räumlichen Eigenschaft insbesondere einer räumlichen Beziehung der biometrischen Vergleichsdaten verglichen werden.

Dieser Verfahrensschritt kann insbesondere dann ausgeführt werden, wenn die biometrischen Daten und die biometrischen Vergleichsdaten jeweils mehr als eine anatomische Landmarke und/oder Oberflächennetz umfassen. Dabei stehen die anatomischen Landmarken und/oder Oberflächennetze der biometrischen Daten untereinander in einer räumlichen Beziehung, die für den in dem medizinischen Bilddatensatz abgebildeten Patienten spezifisch ist. Analog stehen die anatomischen Landmarken und/oder Oberflächennetze der biometrischen Vergleichsdaten untereinander in einer räumlichen Beziehung, die für den in dem medizinischen Vergleichsbilddatensatz abgebildeten Patienten spezifisch ist. Beim Herstellen der Registrierung werden die anatomischen Landmarken und/oder Oberflächennetze der biometrischen Daten und der biometrischen Vergleichsdaten in einen gemeinsamen räumlichen Zusammenhang gebracht.

In Ausführungen der Erfindung ist die Registrierung eine rigide Registrierung. Dabei wird beim Registrieren die räumliche Beziehung der anatomischen Landmarken und/oder Oberflächen der biometrischen Daten und die räumliche Beziehung der anatomischen Landmarken und/oder Oberflächen der biometrischen Vergleichsdaten beibehalten. Mit anderen Worten wird die räumliche Beziehung durch die Registrierung nicht verändert. Bei der rigiden Registrierung werden lediglich der Maßstab der biometrischen Daten und der Maßstab der biometrischen Vergleichsdaten aneinander angepasst.

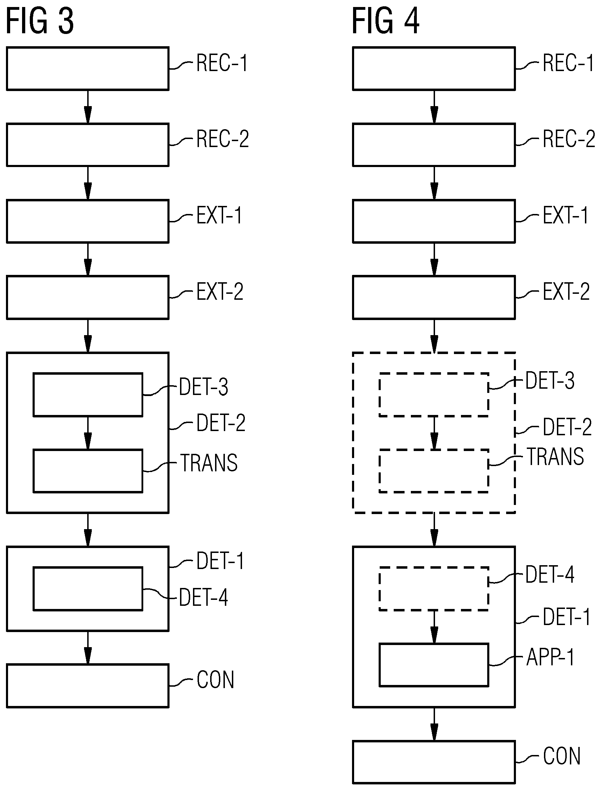

**Figur 3** zeigt ein drittes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten.

Die Verfahrensschritte des Empfangens REC-1 des medizinischen Bilddatensatzes, des Empfangens REC-2 des medizinischen Vergleichsbilddatensatzes, des Extrahierens EXT-1 der biometrischen Daten, des Extrahierens EXT-2 der biometrischen Vergleichsdaten, des Bestimmens DET-1 des Abweichungsmaßes und des Zuordnens CON des medizinischen Bilddatensatzes zu dem medizinischen Vergleichsbilddatensatz sind analog zu der Beschreibung gemäß Figur 1 ausgebildet. Der Verfahrensschritt des Herstellens DET-2 einer Registrierung ist in seiner allgemeinsten Form gemäß der Beschreibung zu Figur 2 ausgebildet.

In dem Ausführungsbeispiel umfasst der Verfahrensschritt des Herstellens DET-2 der Registrierung einen Verfahrensschritt eines Bestimmens DET-3 von einander entsprechenden anatomischen Landmarken und/oder Oberflächennetzen in den biometrischen Daten und den biometrischen Vergleichsdaten und einen Verfahrensschritt eines Transformierens TRANS der einander entsprechenden anatomischen Landmarken und/oder Oberflächennetze in ein gemeinsames Koordinatensystem.

In dem Verfahrensschritt des Bestimmens DET-3 der einander entsprechenden anatomischen Landmarken und/oder Oberflächennetze wird wenigstens ein Paar von zwei anatomischen Landmarken oder zwei Oberflächennetzen gebildet. Dabei entsprechen sich die anatomischen Landmarken und/oder Oberflächennetze eines Paars. Eine anatomische Landmarke bzw. Oberflächennetz des Paars wird von den biometrischen Daten umfasst. Die andere anatomische Landmarke bzw. Oberflächennetz des Paars wird von den biometrischen Vergleichsdaten umfasst. Einander entsprechende anatomische Landmarken bzw. Oberflächennetze betreffen dieselbe Anatomie. Beispielsweise entspricht eine anatomische Landmarke, die den Schwertfortsatz in dem medizinischen Bilddatensatz betrifft einer anatomischen Landmarke, die den Schwertfortsatz in dem medizinischen Vergleichsbilddatensatz betrifft. Analog entspricht ein Oberflächennetz, das die Oberfläche des Herzens in dem medizinischen Bilddatensatz betrifft, einem Oberflächennetz, das die Oberfläche des Herzens in dem medizinischen Vergleichsbilddatensatz betrifft. Insbesondere können mehrere Paare einander entsprechender anatomischer Landmarken und/oder Oberflächennetze bestimmt werden, wenn die biometrischen Daten und die biometrischen Vergleichsdaten mehr als eine anatomische Landmarke und/oder Oberflächennetz umfassen.

In dem Verfahrensschritt des Transformierens TRANS der einander entsprechenden anatomischen Landmarken und/oder Oberflächennetze werden die einander entsprechenden anatomischen Landmarken und/oder Oberflächennetze in ein gemeinsames Koordinatensystem transformiert. Dabei umfassen die wenigstens eine anatomische Landmarke und/oder das wenigstens eine Oberflächennetz der biometrischen Daten und die wenigstens eine anatomische Landmarke und/oder das wenigstens eine Oberflächennetz der biometrischen Vergleichsdaten jeweils eine Koordinate. Die Oberflächennetze können dabei mehr als eine Koordinate umfassen. Wenn die biometrischen Daten und/oder die biometrischen Vergleichsdaten mehr als eine anatomische Landmarke und/oder mehr als ein Oberflächennetz umfassen, wird der Abstand zwischen den anatomischen Landmarken und/oder Oberflächennetzen beim Transformieren TRANS an einen Maßstab des gemeinsamen Koordinatensystems angepasst. Dabei kann der Maßstab auf einer metrischen Einheit basieren. Alternativ kann der Maßstab auf einer anglo-amerikanischen Einheit basieren. Beim Transformieren TRANS werden insbesondere die räumliche Beziehung zwischen den anatomischen Landmarken und/oder Oberflächennetzen der biometrischen Daten und zwischen den anatomischen Landmarken und/oder Oberflächennetzen der biometrischen Vergleichsdaten beibehalten bzw. nicht verändert.

Beim Transformieren TRANS kann ein einander entsprechendes Paar anatomischer Landmarken oder Oberflächennetze aufeinander abgebildet werden. "Aufeinander abbilden" bedeutet, dass die beiden anatomischen Landmarken oder Oberflächennetze auf dieselbe Position in dem gemeinsamen Koordinatensystem positioniert werden. Die anderen anatomischen Landmarken und/oder Oberflächennetze werden dann gemäß ihrer räumlichen Beziehung zu der positionierten anatomischen Landmarke oder Oberflächennetz in dem gemeinsamen Koordinatensystem positioniert.

Alternativ können die anatomischen Landmarken und/oder Oberflächennetze beim Transformieren TRANS derart in dem gemeinsamen Koordinatensystem positioniert werden, dass die quadratische Summe aller Abstände der Positionen der einander entsprechenden anatomischen Landmarken und/oder Oberflächennetze minimal wird. Dabei werden wie oben beschrieben die räumlichen Beziehungen gemäß den biometrischen Daten und den biometrischen Vergleichsdaten beibehalten.

In dem Ausführungsbeispiel umfasst der Verfahrensschritt des Bestimmens DET-1 des Abweichungsmaßes einen Verfahrensschritt eines Bestimmens DET-4 einer Abweichung der Koordinaten der einander entsprechenden Landmarken bzw. Oberflächennetze in dem gemeinsamen Koordinatensystem.

Dabei wird für jedes Paar von einander entsprechenden anatomischen Landmarken bzw. Oberflächennetzen der Abstand zwischen den Positionen der beiden anatomischen Landmarken bzw. Oberflächennetzen bestimmt. Der Abstand der Positionen entspricht dabei der Abweichung der Koordinaten. Der Abstand bzw. die Abweichung wird dabei insbesondere in einer metrischen Einheit oder in einer anglo-amerikanischen Einheit angegeben.

Das Abweichungsmaß kann dabei proportional zu der Summe aller quadratischen Abweichungen bzw. Abstände sein. Das Abweichungsmaß kann alternativ proportional zu der Wurzel aus der Summe aller quadratischen Abweichungen sein. Das Abweichungsmaß kann dabei insbesondere der normierten Wurzel aus der Summe der quadratischen Abstände entsprechen. Der Normierungsfaktor ist dabei die Anzahl an Paaren von einander entsprechenden anatomischen Landmarken bzw. Oberflächennetzen.

Ein Einfluss einer anatomischen Landmarke und/oder eines Oberflächennetzes auf das Abweichungsmaß hängt in Ausführungen der Erfindung durch die zeitliche Stabilität der anatomischen Landmarke bzw. des Oberflächennetzes ab. Dabei hängt die zeitliche Stabilität der anatomischen Landmarke bzw. des Oberflächennetzes von der Anatomie, die sie betrifft ab. Mit anderen Worten ist der Einfluss von zwei einander entsprechenden anatomischen Landmarken bzw. Oberflächennetzen gleich groß. Der Einfluss gibt dabei eine Wichtung der Abweichung zwischen den Koordinaten beim Bestimmen des Abweichungsmaßes vor. Die Wichtung kann dabei für jedes Paar von anatomischen Landmarken und/oder Oberflächennetzen spezifisch sein. Insbesondere werden zeitlich stabile anatomische Landmarken bzw. Oberflächennetze stärker gewichtet als zeitlich instabile Landmarken bzw. Oberflächennetze. Das Abweichungsmaß kann dabei proportional zu der Summe aller gewichteten quadratischen Abweichungen bzw. Abstände sein. Das Abweichungsmaß kann alternativ proportional zu der Wurzel aus der Summe aller gewichteten quadratischen Abweichungen sein. Das Abweichungsmaß kann dabei insbesondere der normierten Wurzel aus der Summe der gewichteten quadratischen Abstände entsprechen.

Eine zeitlich stabile anatomische Landmarke bzw. Oberflächennetz ist räumlich relativ zu dem Patienten zu jedem Zeitpunkt nahezu konstant angeordnet. Die zeitlich stabile anatomische Landmarke beschreibt beispielsweise eine Position eines bestimmten Knochens. Beispielsweise ist eine anatomische Landmarke, die den Schwertfortsatz betrifft zeitlich konstant.

Eine zeitlich instabile anatomische Landmarke ist in ihrer Position relativ zu dem Patienten und insbesondere relativ zu einer zeitlich stabilen anatomischen Landmarke nicht konstant. Eine zeitlich instabile anatomische Landmarke kann beispielsweise eine Apex pulmonis betreffen. Abhängig von einem Atemzustand des ersten oder zweiten Patienten verändert sich die Position der Apex pulmonis. Eine große Abweichung zwischen zwei zeitlich instabilen, einander entsprechenden anatomischen Landmarken, kann entweder darin begründet sein, dass sie unterschiedliche Patienten betreffen oder dass der gleiche Patient zu unterschiedlichen Atemzeitpunkten in dem entsprechenden Bilddatensatz abgebildet wurde.

**Figur 4** zeigt ein viertes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten.

Die Verfahrensschritte des Empfangens REC-1 des medizinischen Bilddatensatzes, des Empfangens REC-2 des medizinischen Vergleichsbilddatensatzes, des Extrahierens EXT-1 der biometrischen Daten, des Extrahierens EXT-2 der biometrischen Vergleichsdaten, des Bestimmens DET-1 des Abweichungsmaßes und des Zuordnens CON des medizinischen Bilddatensatzes zu dem medizinischen Vergleichsbilddatensatz sind analog zu der Beschreibung gemäß Figur 1 ausgebildet. Der optionale Verfahrensschritt des Herstellens DET-2 einer Registrierung ist in seiner allgemeinsten Form gemäß der Beschreibung zu Figur 2 ausgebildet. Die optionalen Verfahrensschritte des Bestimmens DET-3 von einander entsprechenden Landmarken und/oder Oberflächennetzen, des Transformierens TRANS der einander entsprechenden Landmarken und/oder Oberflächennetze und des Bestimmens DET-4 einer Abweichung der Koordinaten der einander entsprechenden Landmarken bzw. Oberflächennetze sind gemäß der Beschreibung zu Figur 3 ausgebildet.

Das Verfahren umfasst außerdem einen Verfahrensschritt eines Anwendens APP-1 einer trainierten Funktion auf die biometrischen Vergleichsdaten und die biometrischen Daten. Dabei wird das Abweichungsmaß bestimmt.

In Ausführungen der Erfindung wurden die biometrischen Daten und die biometrischen Vergleichsdaten gemäß einer der Ausführungen des Verfahrensschrittes des Herstellens DET-2 einer Registrierung registriert. In einer alternativen Ausführung der Erfindung wird der Verfahrensschritt des Herstellens DET-2 einer Registrierung nicht ausgeführt. In einer alternativen Ausführung umfasst der Verfahrensschritt des Anwendens APP-1 der trainierten Funktion den Verfahrensschritt des Herstellens DET-2 einer Registrierung in einer seiner Ausführungen.

Der Verfahrensschritt des Bestimmens DET-4 der Abweichung der Koordinaten wird optional ausgeführt.

Das Abweichungsmaß wird durch das Anwenden APP-1 der trainierten Funktion auf die biometrischen Daten und auf die biometrischen Vergleichsdaten bestimmt. Dabei kann die trainierte Funktion insbesondere die räumliche Beziehung der anatomischen Landmarken und/oder Oberflächennetze der biometrischen Daten mit der räumlichen Beziehung der anatomischen Landmarken und/oder Oberflächennetze der biometrischen Vergleichsdaten vergleichen. Darauf basierend kann durch das Anwenden APP-1 der trainierten Funktion das Abweichungsmaß bestimmt werden, welches angibt, mit welcher Wahrscheinlichkeit die biometrischen Daten und die biometrischen Vergleichsdaten zwei unterschiedliche Patienten beschreiben bzw. betreffen.

Analog zu der Beschreibung zu Figur 3 kann ein Einfluss der anatomischen Landmarken bzw. Oberflächennetze von ihrer zeitlichen Stabilität abhängen. Die trainierte Funktion kann dabei eine anatomische Landmarke und/oder ein Oberflächennetz beim Bestimmen DET-1 des Abweichungsmaßes in Abhängigkeit ihrer zeitlichen Stabilität gewichten. Je größer die zeitliche Stabilität, desto größer die Gewichtung, desto größer der Einfluss auf das Abweichungsmaß.

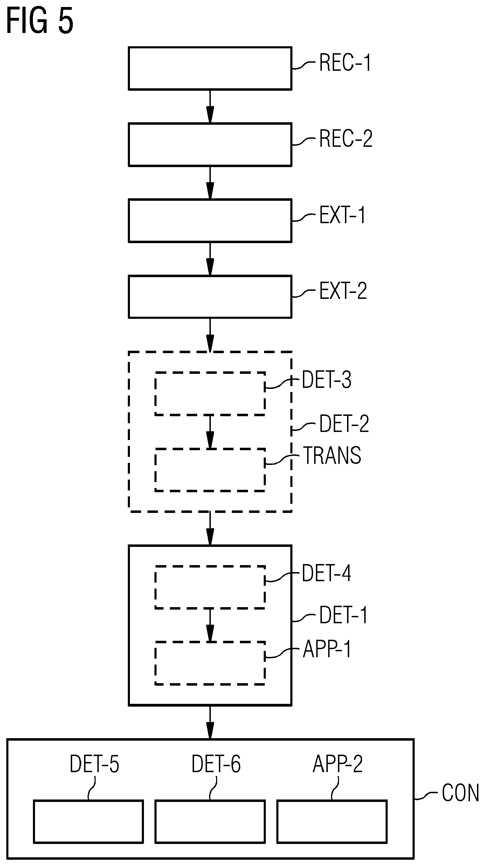

**Figur 5** zeigt ein fünftes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten.

Die Verfahrensschritte des Empfangens REC-1 des medizinischen Bilddatensatzes, des Empfangens REC-2 des medizinischen Vergleichsbilddatensatzes, des Extrahierens EXT-1 der biometrischen Daten, des Extrahierens EXT-2 der biometrischen Vergleichsdaten, des Bestimmens DET-1 des Abweichungsmaßes und des Zuordnens CON des medizinischen Bilddatensatzes zu dem medizinischen Vergleichsbilddatensatz sind analog zu der Beschreibung gemäß Figur 1 ausgebildet. Der optionale Verfahrensschritt des Herstellens DET-2 einer Registrierung ist in seiner allgemeinsten Form gemäß der Beschreibung zu Figur 2 ausgebildet. Die optionalen Verfahrensschritte des Bestimmens DET-3 von einander entsprechenden Landmarken und/oder Oberflächennetzen, des Transformierens TRANS der einander entsprechenden Landmarken und/oder Oberflächennetze und des Bestimmens DET-4 einer Abweichung der Koordinaten der einander entsprechenden Landmarken bzw. Oberflächennetze sind gemäß der Beschreibung zu Figur 3 ausgebildet. Der optionale Verfahrensschritt des Anwendens APP-1 einer trainierten Funktion ist gemäß der Beschreibung zu Figur 4 ausgebildet.

Der Verfahrensschritt des Zuordnens CON des medizinischen Bilddatensatzes zu dem medizinischen Vergleichsbilddatensatz umfasst wenigstens einen der folgenden Verfahrensschritte:
- Erstellen DET-5 einer Verknüpfung zwischen dem medizinischen Bilddatensatz und dem medizinischen Vergleichsbilddatensatz in einer Datenbank;
- Bestimmen DET-6 einer gemeinsamen Identifikationsnummer für den medizinischen Bilddatensatz und den medizinischen Vergleichsbilddatensatz;
- Anwenden APP-2 einer Funktion zur Bildanalyse auf den medizinischen Vergleichsbilddatensatz und den medizinischen Bilddatensatz.

Mit anderen Worten wird wenigstens einer der genannten Verfahrensschritte dann ausgeführt, wenn davon ausgegangen werden kann, dass der medizinische Bilddatensatz und der medizinische Vergleichsbilddatensatz denselben, insbesondere den ersten Patienten, abbilden. Wenn das Abstandsmaß den Schwellenwert unterschreitet, kann davon ausgegangen werden, dass der medizinische Bilddatensatz und der medizinische Vergleichsbilddatensatz denselben Patienten abbilden.

In dem Verfahrensschritt des Erstellens DET-5 der Verknüpfung zwischen dem medizinischen Bilddatensatz und dem medizinischen Vergleichsbilddatensatz werden der medizinische Bilddatensatz und der medizinische Vergleichsbilddatensatz in einer Datenbank verknüpft. Dabei sind der medizinische Bilddatensatz und der medizinische Vergleichsbilddatensatz in der Datenbank hinterlegt. Die Datenbank kann dabei insbesondere ein PACS, ein KIS oder ein RIS sein. Die Verknüpfung ist dabei Link bzw. ein Verweis auf einen Speicherort in der Datenbank. Beim Erstellen der Verknüpfung wird somit dem medizinischen Bilddatensatz ein Link zu dem Speicherort des medizinischen Vergleichsbilddatensatzes in der Datenbank hinzugefügt. Alternativ oder zusätzlich wird beim Erstellen der Verknüpfung wird dem medizinischen Vergleichsbilddatensatz ein Link zu dem Speicherort des medizinischen Bilddatensatzes in der Datenbank hinzugefügt. Bei einem Abrufen bzw. Aufrufen des medizinischen Bilddatensatzes oder des medizinischen Vergleichsbilddatensatzes durch einen Nutzer oder durch eine Applikation bzw. Anwendung kann automatisch über den Link bzw. über die Verknüpfung auch der jeweils andere Bilddatensatz aufgerufen werden.

In dem Verfahrensschritt DET-6 des Bestimmens einer gemeinsamen Identifikationsnummer (Akronym: ID) wird für den medizinischen Bilddatensatz und den medizinischen Vergleichsbilddatensatz eine gemeinsame ID bestimmt. Die gemeinsame ID kann dann dem medizinischen Bilddatensatz und dem medizinischen Vergleichsbilddatensatz zugeordnet werden. Die gemeinsame ID ist dabei eindeutig für die Gruppe der einander zugeordneten Bilddatensätze. Wenn der medizinische Bilddatensatz und der medizinische Vergleichsbilddatensatz in der Datenbank gespeichert sind, können über eine Datenbankabfrage nach der gemeinsamen ID der medizinische Bilddatensatz und der medizinische Vergleichsbilddatensatz bereitgestellt werden. Alle anderen dem medizinischen Bilddatensatz bzw. dem medizinischen Vergleichsbilddatensatz zugeordneten Bilddatensätze können ebenfalls die gemeinsame ID umfassen und werden bei der Datenbankabfrage bereitgestellt. Somit werden alle Bilddatensätze, die einen Patienten betreffen durch eine einzige Datenbankabfrage bereitgestellt. Dabei ist eine Identifizierung des Patienten nicht notwendig. Die gemeinsame ID ist unabhängig von möglicherweise identifizierbaren Patientendaten. Die gemeinsame ID kann dabei beispielsweise eine generische Ziffern- und/oder Zahlenfolge sein.

In dem Verfahrensschritt des Anwendens APP-2 einer Funktion zur Bildanalyse wird die Funktion zur Bildanalyse auf den medizinischen Bilddatensatz und den medizinischen Vergleichsbilddatensatz angewendet, wenn das Abweichungsmaß den Schwellenwert unterschreitet, wenn also der medizinische Bilddatensatz und der medizinische Vergleichsbilddatensatz denselben Patienten, insbesondere den ersten Patienten, abbilden. Die Funktion zur Bildanalyse kann insbesondere dazu ausgebildet sein, das medizinische Bild des medizinischen Bilddatensatzes mit dem medizinischen Bild des medizinischen Vergleichsbilddatensatzes zu vergleichen. Alternativ oder zusätzlich kann die Funktion zur Bildanalyse dazu ausgebildet sein, die beiden medizinischen Bilder zu registrieren.

In Ausführungen der Erfindung wurde der medizinische Vergleichsbilddatensatz zeitlich vor dem medizinischen Bilddatensatz erfasst. Durch das Anwenden APP-2 der Funktion zur Bildanalyse wird eine zeitliche Veränderung zwischen dem medizinischen Vergleichsbilddatensatz und dem medizinischen Bilddatensatz bestimmt. Mit anderen Worten ist die Funktion zur Bildanalyse für eine longitudinale bzw. zeitliche Analyse von Bilddaten ausgebildet. Insbesondere kann die Funktion im Zuge einer Follow-Up Untersuchung bzw. einer Folge-Untersuchung eines Patienten, beispielsweise des ersten Patienten, angewendet werden. Beispielsweise kann durch das Anwenden APP-2 der Funktion zur Bildanalyse eine Veränderung einer Läsion beobachtet bzw. analysiert werden. Die Läsion kann beispielsweise ein Tumor und/oder eine Veränderung eines Gewebes und/oder ein Aneurysma und/oder eine Arteriosklerose etc. sein. Die Funktion zur Bildanalyse kann insbesondere dazu ausgebildet sein, neu auftretende Läsionen in dem medizinischen Bilddatensatz zu erkennen. Alternativ oder zusätzlich kann die Funktion zur Bildanalyse dazu ausgebildet sein, zu erkennen, wenn eine Läsion mit der Zeit verschwindet. Der Nutzer kann über die Veränderung zwischen dem medizinischen Bilddatensatz und dem medizinischen Vergleichsbilddatensatz informiert werden. Beispielsweise kann er durch eine Bildschirmausgabe und/oder durch eine Ausgabe in einem Patientenreport über die Veränderung informiert werden.

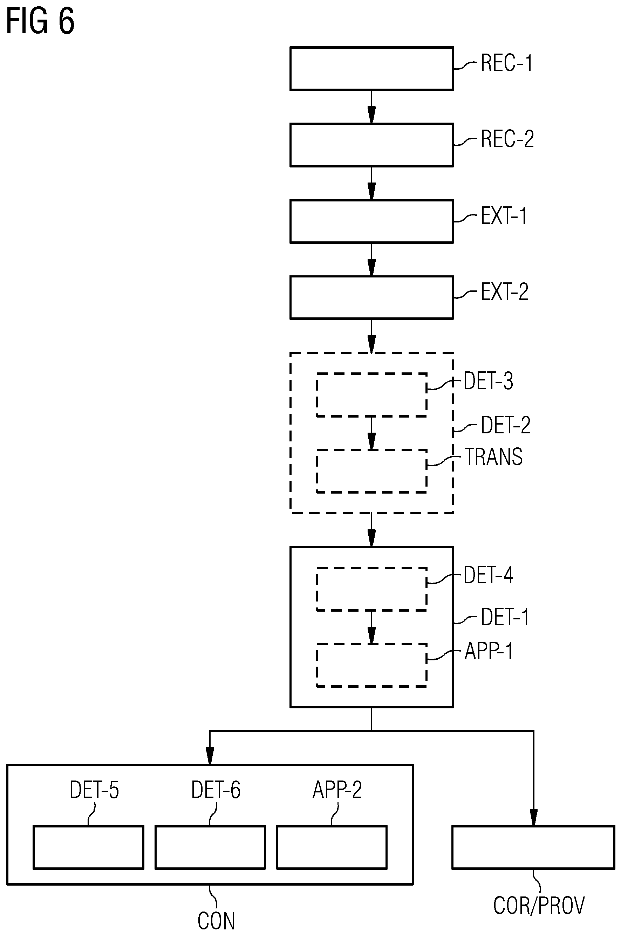

**Figur 6** zeigt ein sechstes Ausführungsbeispiel eines computerimplementierten Verfahrens zum Zuordnen eines medizinischen Bilddatensatzes zu einem medizinischen Vergleichsbilddatensatz in Abhängigkeit von biometrischen Daten.

Die Verfahrensschritte des Empfangens REC-1 des medizinischen Bilddatensatzes, des Empfangens REC-2 des medizinischen Vergleichsbilddatensatzes, des Extrahierens EXT-1 der biometrischen Daten, des Extrahierens EXT-2 der biometrischen Vergleichsdaten, des Bestimmens DET-1 des Abweichungsmaßes und des Zuordnens CON des medizinischen Bilddatensatzes zu dem medizinischen Vergleichsbilddatensatz sind analog zu der Beschreibung gemäß Figur 1 ausgebildet. Der optionale Verfahrensschritt des Herstellens DET-2 einer Registrierung ist in seiner allgemeinsten Form gemäß der Beschreibung zu Figur 2 ausgebildet. Die optionalen Verfahrensschritte des Bestimmens DET-3 von einander entsprechenden Landmarken und/oder Oberflächennetzen, des Transformierens TRANS der einander entsprechenden Landmarken und/oder Oberflächennetze und des Bestimmens DET-4 einer Abweichung der Koordinaten der einander entsprechenden Landmarken bzw. Oberflächennetze sind gemäß der Beschreibung zu Figur 3 ausgebildet. Der optionale Verfahrensschritt des Anwendens APP-1 einer trainierten Funktion ist gemäß der Beschreibung zu Figur 4 ausgebildet. Die Verfahrensschritte des Erstellens DET-5 der Verknüpfung, des Bestimmens DET-6 der gemeinsamen Identifikationsnummer und des Anwendens APP-2 der Funktion zur Bildanalyse sind gemäß der Beschreibung zu Figur 5 ausgebildet.

Das Verfahren umfasst außerdem einen Verfahrensschritt eines Korrigierens COR einer vorläufigen Zuordnung und/oder eines Bereitstellens PROV eines Warnsignals, wenn das Abweichungsmaß größer oder gleich dem Schwellenwert ist.

In einer Ausführung der Erfindung sind der medizinische Bilddatensatz und der medizinische Vergleichsbilddatensatz durch eine vorläufige Zuordnung einander zugeordnet. Die vorläufige Zuordnung kann dabei in Form einer vorläufigen Verknüpfung und/oder einer vorläufigen Identifikationsnummer (Akronym: ID) realisiert sein. Die vorläufige Verknüpfung und die vorläufige ID sind dabei gemäß der Beschreibung zu der Verknüpfung und der gemeinsamen ID zu Figur 5 ausgebildet.

Wenn das Abweichungsmaß größer oder gleich dem Schwellenwert ist, wird die vorläufige Zuordnung in dem Verfahrensschritt des Korrigierens COR der vorläufigen Zuordnung korrigiert.

Das Korrigieren COR der vorläufigen Zuordnung umfasst dabei in Ausführungen der Erfindung ein Löschen der vorläufigen Zuordnung. Mit anderen Worten umfasst das Korrigieren COR der vorläufigen Zuordnung dann ein Löschen der Verknüpfung in dem medizinischen Bilddatensatz und dem medizinischen Vergleichsbilddatensatz. Alternativ oder zusätzlich umfasst das Korrigieren COR der vorläufigen Zuordnung ein Löschen der vorläufigen ID in dem medizinischen Bilddatensatz und/oder dem medizinischen Vergleichsbilddatensatz.

Alternativ oder zusätzlich umfasst das Korrigieren COR der vorläufigen Zuordnung ein Bereitstellen einer Korrekturinformation für den Nutzer. Die Korrekturinformation wird dabei dem Nutzer mit einer Anzeigeeinheit angezeigt. Die Anzeigeeinheit kann insbesondere ein Bildschirm bzw. ein Monitor sein. Die Korrekturinformation umfasst eine Information darüber, dass das Abweichungsmaß größer oder gleich dem Schwellenwert ist. Insbesondere weist die Korrekturinformation den Nutzer darauf hin, dass die vorläufige Zuordnung vermutlich falsch ist. Der Nutzer kann die Korrekturinformation über eine Nutzereingabe bestätigen oder verwerfen. Bestätigt der Nutzer die Korrekturinformation wird eine Bestätigungsinformation empfangen. Das Empfangen der Bestätigungsinformation kann dabei ein Löschen der vorläufigen Zuordnung bewirken. Verwirft der Nutzer die Korrekturinformation, wird eine Verwerfungsinformation empfangen. Dann kann die vorläufige Zuordnung beibehalten werden. Der Nutzer kann die Korrekturinformation insbesondere dann verwerfen, wenn der medizinische Bilddatensatz und der medizinische Vergleichsbilddatensatz denselben Patienten abbilden. Das Abweichungsmaß kann dann trotzdem größer oder gleich dem Schwellenwert sein, wenn sich der Patient zwischen den beiden Aufnahmen des medizinischen Bilddatensatzes und des medizinischen Vergleichsbilddatensatzes beispielsweise durch eine starke Gewichtsveränderung stark verändert hat. Die Nutzereingabe kann insbesondere über eine Eingabeeinheit empfangen werden. Die Eingabeeinheit kann beispielsweise eine Tastatur, ein berührungsempfindlicher Bildschirm (engl. Touchscreen), ein berührungsempfindliches Tastfeld (engl. Touchpad) und/oder eine Computer-Maus etc. sein.

Alternativ oder zusätzlich zu dem Verfahrensschritt des Korrigierens COR der vorläufigen Zuordnung kann das Verfahren den Verfahrensschritt des Bereitstellens PROV eines Warnsignals umfassen. Das Warnsignal wird dann bereitgestellt, wenn das Abweichungsmaß den Schwellenwert unterschreitet. Das Warnsignal kann dabei optisch und/oder akustisch sein.

Das Warnsignal kann dabei insbesondere die oben beschriebene Korrekturinformation sein. Die Korrekturinformation kann mittels der Anzeigeeinheit bereitgestellt werden. Alternativ kann die Korrekturinformation auch mit einem Lautsprecher bereitgestellt werden. Dann kann die Korrekturinformation in Form einer Durchsage bereitgestellt werden.

Alternativ oder zusätzlich kann das Warnsignal ein Warnton bzw. ein akustisches Signal sein. Das akustische Signal kann dabei insbesondere mit einem Lautsprecher bereitgestellt werden.

Alternativ oder zusätzlich kann das Warnsignal ein Aufleuchten beispielsweise ein Blinken einer Warnlampe sein. Alternativ kann das Warnsignal ein Umschalten einer Ampel beispielsweise von "Grün" auf "Rot" sein.

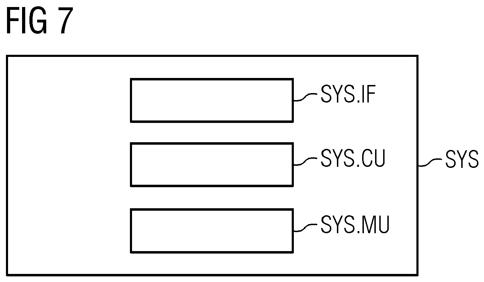

**Figur 7** zeigt ein Zuordnungssystem SYS zum Zuordnen eines medizinischen Bilddatensatzes zu einem medizinischen Vergleichsbilddatensatz in Abhängigkeit von biometrischen Daten.

Das dargestellte Zuordnungssystem SYS zum Zuordnen eines medizinischen Bilddatensatzes zu einem medizinischen Vergleichsbilddatensatz in Abhängigkeit von biometrischen Daten ist dazu ausgebildet ein erfindungsgemäßes Verfahren zum Zuordnen eines medizinischen Bilddatensatzes zu einem medizinischen Vergleichsbilddatensatz in Abhängigkeit von biometrischen Daten auszuführen. Das Zuordnungssystem SYS umfasst eine Schnittstelle SYS.IF, eine Recheneinheit SYS.CU und eine Speichereinheit SYS.MU.

Das Zuordnungssystem SYS kann insbesondere ein Computer, ein Mikrocontroller oder ein integrierter Schaltkreis (integrated circuit, IC) sein. Alternativ kann das Zuordnungssystem SYS ein reales oder virtuelles Computer-Netzwerk sein (eine technische Bezeichnung für ein reales Computer-Netzwerk ist "Cluster", eine technische Bezeichnung für ein virtuelles Computer-Netzwerk ist "Cloud"). Das Zuordnungssystem SYS kann als virtuelles System ausgebildet sein, welches auf einem Computer oder einem realen Computer-Netzwerk oder einem virtuellen Computer-Netzwerk ausgeführt wird (eine technische Bezeichnung ist "Virtualization").

Die Schnittstelle SYS.IF kann eine Hardware- oder Software-Schnittstelle sein (beispielsweise ein PCI bus, USB oder Firewire). Die Recheneinheit SYS.CU kann Hardware und/oder Software Bestandteile umfassen, beispielsweise einen Mikroprozessor oder einen sogenannten FPGA (Field Programmable Gate Way). Die Speichereinheit SYS.MU kann als nicht permanent arbeitender Arbeitsspeicher (Random Access Memory, RAM) oder als permanenter Massenspeicher (Festplatte, USB-Stick, SD-Karte, Solid State Disk (SSD)) ausgebildet sein.

Die Schnittstelle SYS.IF kann insbesondere eine Mehrzahl an Sub-Schnittstellen umfassen, die unterschiedliche Verfahrensschritte des jeweiligen erfindungsgemäßen Verfahrens ausführen. Mit anderen Worten kann die Schnittstelle SYS.IF als eine Mehrzahl an Schnittstellen SYS.IF ausgebildet sein. Die Recheneinheit SYS.CU kann insbesondere eine Mehrzahl an Sub-Recheneinheiten umfassen, die unterschiedliche Verfahrensschritte des jeweiligen erfindungsgemäßen Verfahrens ausführen. Mit anderen Worten kann die Recheneinheit SYS.CU als eine Mehrzahl an Recheneinheiten SYS.CU.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Computerimplementiertes Verfahren zum Zuordnen eines medizinischen Bilddatensatzes zu einem medizinischen Vergleichsbilddatensatz in Abhängigkeit von biometrischen Daten umfassend folgende Verfahrensschritte:
- Empfangen (REC-1) des medizinischen Bilddatensatzes,
wobei der medizinische Bilddatensatz ein medizinisches Bild eines Patienten umfasst,
- Empfangen (REC-2) wenigstens eines medizinischen Vergleichsbilddatensatzes,
wobei der medizinische Vergleichsbilddatensatz ein medizinisches Bild des Patienten oder eines anderen Patienten umfasst,
- Extrahieren (EXT-1) von biometrischen Daten basierend auf dem medizinischen Bilddatensatz,
wobei die biometrischen Daten eine anatomische Ausprägung des Patienten, dessen medizinisches Bild von dem medizinischen Bilddatensatz umfasst ist, beschreiben,
- Extrahieren (EXT-2) von biometrischen Vergleichsdaten basierend auf dem medizinischen Vergleichsbilddatensatz, wobei die biometrischen Vergleichsdaten eine anatomische Ausprägung des Patienten, dessen medizinisches Bild von dem medizinischen Vergleichsbilddatensatz umfasst ist, beschreiben,
- Bestimmen (DET-1) eines Abweichungsmaßes zwischen den biometrischen Vergleichsdaten und den biometrischen Daten, wobei das Abweichungsmaß beschreibt, wie stark die biometrischen Daten von den biometrischen Vergleichsdaten abweichen,
- Zuordnen (CON) des medizinischen Bilddatensatzes zu dem medizinischen Vergleichsbilddatensatz, wenn das Abweichungsmaß einen Schwellenwert unterschreitet.

2. Verfahren nach Anspruch 1,
wobei der medizinische Vergleichsbilddatensatz wenigstens einen Körperteil eines ersten Patienten abbildet und/oder wobei der medizinische Bilddatensatz wenigstens einen Körperteil des ersten Patienten oder eines zweiten Patienten abbildet.

3. Verfahren nach Anspruch 2,
wobei die biometrischen Daten wenigstens eine anatomische Landmarke und/oder wenigstens ein Oberflächennetz wenigstens einer Anatomie des abgebildeten Körperteils in dem medizinischen Bilddatensatz umfassen,
wobei die biometrischen Vergleichsdaten wenigstens eine anatomische Landmarke und/oder wenigstens ein Oberflächennetz wenigstens einer Anatomie des abgebildeten Körperteils in dem medizinischen Vergleichsbilddatensatz umfassen.

4. Verfahren nach Anspruch 3,
wobei die anatomische Landmarke und/oder das Oberflächennetz wenigstens eine Koordinate in dem medizinischen Bilddatensatz bzw. in dem medizinischen Vergleichsbilddatensatz umfasst.

5. Verfahren nach einem der vorhergehenden Ansprüche, weiterhin umfassend folgenden Verfahrensschritt:
- Herstellen (DET-2) einer Registrierung zwischen den biometrischen Vergleichsdaten und den biometrischen Daten,
wobei der Verfahrensschritt des Bestimmens (DET-1) des Abweichungsmaßes basierend auf der Registrierung erfolgt.

6. Verfahren nach Anspruch 4 und 5,
wobei der Verfahrensschritt des Herstellens (DET-2) der Registrierung folgende Verfahrensschritte umfasst:
- Bestimmen (DET-3) von einander entsprechenden anatomischen Landmarken und/oder Oberflächennetzen in den biometrischen Vergleichsdaten und den biometrischen Daten,
- Transformieren (TRANS) der einander entsprechenden anatomischen Landmarken und/oder Oberflächennetze in ein gemeinsames Koordinatensystem,
wobei der Verfahrensschritt des Bestimmens (DET-1) des Abweichungsmaßes folgenden Verfahrensschritt umfasst:
- Bestimmen (DET-4) einer Abweichung der Koordinaten der einander entsprechenden anatomischen Landmarken bzw. Oberflächennetze in dem gemeinsamen Koordinatensystem.

7. Verfahren nach einem der Ansprüche 5 oder 6,
wobei das Herstellen (DET-2) der Registrierung der biometrischen Vergleichsdaten und der biometrischen Daten eine rigide Registrierung umfasst.

8. Verfahren nach einem der vorhergehenden Ansprüche,
wobei der Verfahrensschritt des Bestimmens (DET-1) des Abweichungsmaßes folgenden Verfahrensschritt umfasst:
- Anwenden (APP-1) einer trainierten Funktion auf die biometrischen Vergleichsdaten und die biometrischen Daten, wodurch das Abweichungsmaß bestimmt wird.

9. Verfahren nach einem der Ansprüche 5 bis 8 in Kombination mit Anspruch 3, wobei ein Einfluss der wenigstens einen anatomischen Landmarke und/oder des wenigstens einen Oberflächennetzes auf das Abweichungsmaß von ihrer zeitlichen Stabilität abhängt.

10. Verfahren nach einem der vorhergehenden Ansprüche,
wobei der Verfahrensschritt des Zuordnens (CON) des medizinischen Bilddatensatzes wenigstens einen der folgenden Schritte umfasst:
- Erstellen (DET-5) einer Verknüpfung zwischen dem medizinischen Bilddatensatz und dem medizinischen Vergleichsbilddatensatz in einer Datenbank;
- Bestimmen (DET-6) einer gemeinsamen Identifikationsnummer für den medizinischen Bilddatensatz und den medizinischen Vergleichsbilddatensatz;
- Anwenden (APP-2) einer Funktion zur Bildanalyse auf den medizinischen Vergleichsbilddatensatz und den medizinischen Bilddatensatz.

11. Verfahren nach Anspruch 10,
wobei der medizinische Vergleichsbilddatensatz zeitlich vor dem medizinischen Bilddatensatz erfasst wurde,
wobei durch das Anwenden (APP-2) der Funktion zur Bildanalyse auf den medizinischen Vergleichsbilddatensatz und den medizinischen Bilddatensatz eine zeitliche Veränderung zwischen dem medizinischen Vergleichsbilddatensatz und dem medizinischen Bilddatensatz bestimmt wird.

12. Verfahren nach einem der vorhergehenden Ansprüche,
wobei der medizinische Bilddatensatz dem medizinischen Vergleichsbilddatensatz durch eine vorläufige Zuordnung zugeordnet ist,
wobei das Verfahren weiterhin folgenden Verfahrensschritt umfasst:
- Korrigieren (COR) der vorläufigen Zuordnung zwischen dem medizinischen Bilddatensatz und dem medizinischen Vergleichsbilddatensatz, wenn das Abweichungsmaß größer oder gleich dem Schwellenwert ist.

13. Verfahren nach einem der vorhergehenden Ansprüche,
wobei das Verfahren weiterhin folgenden Verfahrensschritt umfasst:
- Bereitstellen (PROV) eines Warnsignals, wenn das Abweichungsmaß größer oder gleich dem Schwellenwert ist.

14. Zuordnungssystem (SYS) zum Zuordnen eines medizinischen Bilddatensatzes zu einem medizinischen Vergleichsbilddatensatz in Abhängigkeit von biometrischen Daten umfassend eine Schnittstelle (SYS.IF) und eine Recheneinheit (SYS.CU),
wobei die Schnittstelle (SYS.IF) zum Empfangen (REC-1) des medizinischen Bilddatensatzes ausgebildet ist,
wobei der medizinische Bilddatensatz ein medizinisches Bild eines Patienten umfasst,
wobei die Schnittstelle (SYS.IF) weiterhin zum Empfangen (REC-2) wenigstens eines medizinischen Vergleichsbilddatensatzes ausgebildet ist,
wobei der medizinische Vergleichsbilddatensatz ein medizinisches Bild des Patienten oder eines anderen Patienten umfasst,
wobei die Recheneinheit (SYS.CU) zum Extrahieren (EXT-1) von biometrischen Daten basierend auf dem medizinischen Bilddatensatz ausgebildet ist,
wobei die biometrischen Daten eine anatomische Ausprägung des Patienten, dessen medizinisches Bild von dem medizinischen Bilddatensatz umfasst ist, beschreiben,
wobei die Recheneinheit (SYS.CU) weiterhin zum Extrahieren (EXT-2) von biometrischen Vergleichsdaten basierend auf dem medizinischen Vergleichsbilddatensatz ausgebildet ist,
wobei die biometrischen Vergleichsdaten eine anatomische Ausprägung des Patienten, dessen medizinisches Bild von dem medizinischen Vergleichsbilddatensatz umfasst ist, beschreiben, wobei die Recheneinheit (SYS.CU) weiterhin zum Bestimmen (DET-1) eines Abweichungsmaßes zwischen den biometrischen Vergleichsdaten und den biometrischen Daten ausgebildet ist, wobei das Abweichungsmaß beschreibt, wie stark die biometrischen Daten von den biometrischen Vergleichsdaten abweichen, wobei die Recheneinheit (SYS.CU) weiterhin zum Zuordnen (CON) des medizinischen Bilddatensatzes zu dem medizinischen Vergleichsbilddatensatz ausgebildet ist, wenn das Abweichungsmaß einen Schwellenwert unterschreitet.

15. Computerprogramm oder computerlesbares Speichermedium umfassend Programmabschnitte, um alle Schritte des Verfahrens nach einem der Ansprüche 1 bis 13 auszuführen, wenn die Programmabschnitte von dem Zuordnungssystem (SYS) ausgeführt werden.

## Claims

1. Computer-implemented method for assigning a medical image dataset to a medical comparison image dataset depending on biometric data, comprising the following method steps:
- Receiving (REC-1) the medical image dataset, wherein the medical image dataset comprises a medical image of a patient,
- Receiving (REC-2) at least one medical comparison image dataset,
wherein the medical comparison image dataset comprises a medical image of the patient or another patient,
- Extracting (EXT-1) biometric data based on the medical image dataset,
wherein the biometric data describes an anatomical characteristic of the patient, the medical image of which is included in the medical image dataset,
- Extracting (EXT-2) biometric comparison data based on the medical comparison image dataset,
wherein the biometric comparison data describes an anatomical characteristic of the patient, the medical image of which is included in the medical comparison image dataset,
- Determining (DET-1) a measure of difference between the biometric comparison data and the biometric data,
wherein the measure of difference describes how significantly the biometric data differs from the biometric comparison data,
- Assigning (CON) the medical image dataset to the medical comparison image dataset when the measure of difference does not exceed a threshold value.

2. Method according to claim 1,
wherein the medical comparison image dataset maps at least one part of the body of a first patient and/or
wherein the medical image dataset maps at least one part of the body of the first patient or a second patient.

3. Method according to claim 2,
wherein the biometric data comprises at least one anatomical landmark and/or at least one surface network of at least one anatomy of the mapped part of the body in the medical image dataset,
wherein the biometric comparison data comprises at least one anatomical landmark and/or at least one surface network of at least one anatomy of the mapped part of the body in the medical comparison image dataset.

4. Method according to claim 3,
wherein the anatomical landmark and/or the surface network comprises at least one coordinate in the medical image dataset or in the medical comparison image dataset.

5. Method according to one of the preceding claims, furthermore comprising the following method step:
- Establishing (DET-2) a registration between the biometric comparison data and the biometric data,
wherein the method step of determining (DET-1) the measure of difference is undertaken based on the registration.

6. Method according to claim 4 and 5,
wherein the method step of establishing (DET-2) the registration comprises the following method steps:
- Determining (DET-3) anatomical landmarks and/or surface networks corresponding to one another in the biometric comparison data and the biometric data,
- Transforming (TRANS) the anatomical landmarks and/or surface networks corresponding to one another into a common coordinate system,
wherein the method step of determining (DET-1) the measure of difference comprises the following method step:
- Determining (DET-4) a difference of the coordinates of the anatomical landmarks or surface networks corresponding to one another in the common coordinate system.

7. Method according to one of claims 5 or 6,
wherein the establishing (DET-2) of the registration of the biometric comparison data and of the biometric data comprises a rigid registration.

8. Method according to one of the preceding claims,
wherein the method step of determining (DET-1) the measure of difference comprises the following method step:
- Applying (APP-1) a trained function to the biometric comparison data and the biometric data,
whereby the measure of difference is determined.

9. Method according to one of claims 5 to 8 in combination with claim 3, wherein an influence of the at least one anatomical landmark and/or the at least one surface network on the measure of difference depends on their temporal stability.

10. Method according to one of the preceding claims,
wherein the method step of assigning (CON) the medical image dataset comprises at least one of the following steps:
- Creating (DET-5) a linkage between the medical image dataset and the medical comparison image dataset in a database;
- Determining (DET-6) a common identification number for the medical image dataset and the medical comparison image dataset;
- Applying (APP-2) a function for image analysis to the medical comparison image dataset and the medical image dataset.

11. Method according to claim 10,
wherein the medical comparison image dataset has been acquired at a time before the medical image dataset,
wherein, by the application (APP-2) of the function for image analysis to the medical comparison image dataset and the medical image dataset, a temporal change between the medical comparison image dataset and the medical image dataset is determined.

12. Method according to one of the preceding claims,
wherein the medical image dataset is assigned to the medical comparison image dataset by a provisional assignment,
wherein the method furthermore comprises the following method step:
- Correcting (COR) the provisional assignment between the medical image dataset and the medical comparison image dataset when the measure of difference is greater than or equal to the threshold value.

13. Method according to one of the preceding claims,
wherein the method furthermore comprises the following method step:
- Provision (PROV) of a warning signal, when the measure of difference is greater than or equal to threshold value.

14. Assignment system (SYS) for assigning a medical image dataset to a medical comparison image dataset depending on biometric data comprising an interface (SYS.IF) and a computing unit (SYS.CU),
wherein the interface (SYS.IF) is embodied for receiving (REC-1) the medical image dataset,
wherein the medical image dataset comprises a medical image of a patient,
wherein the interface (SYS.IF) is furthermore embodied for receiving (REC-2) at least one medical comparison image dataset,
wherein the medical comparison image dataset comprises a medical image of the patient or another patient,
wherein the computing unit (SYS.CU) is embodied for extracting (EXT-1) biometric data based on the medical image dataset, wherein the biometric data describes an anatomical characteristic of the patient, the medical image of which is included in the medical image dataset,
wherein the computing unit (SYS.CU) is furthermore embodied for extracting (EXT-2) biometric comparison data based on the medical comparison image dataset,
wherein the biometric comparison data describes an anatomical characteristic of the patient, the medical image of which is included in the medical comparison image dataset,
wherein the computing unit (SYS.CU) is furthermore embodied for determining (DET-1) a measure of difference between the biometric comparison data and the biometric data,
wherein the measure of difference describes how significantly the biometric data differs from the biometric comparison data, wherein the computing unit (SYS.CU) is furthermore embodied for assigning (CON) the medical image dataset to the medical comparison image dataset when the measure of difference does not exceed a threshold value.

15. Computer program or computer-readable storage medium comprising program sections for carrying out all steps of the method according to one of claims 1 to 13 when the program sections are executed by the assignment system (SYS).

## Revendications

1. Procédé mis en œuvre par ordinateur d'affectation d'un ensemble de données d'image médicale comparatives en fonction de données biométriques comprenant les stades de procédé suivants :
- réception (REC-1) de l'ensemble de données d'image médicale, dans lequel l'ensemble de données d'image médicale comprend une image médicale d'un patient,
- réception (REC-2) d'au moins un ensemble de données d'image médicale comparatives,
dans lequel l'ensemble de données d'image médicale comparatives comprend une image médicale du patient ou d'un autre patient,
- extraction (EXT-1) de données biométriques reposant sur l'ensemble de données d'image médicale,
dans lequel les données biométriques décrivent une empreinte anatomique du patient, dont l'image médicale est comprise par l'ensemble de données d'image médicale,
- extraction (EXT-2) de données biométriques comparatives reposant sur l'ensemble de données d'image médicale comparatives,
dans lequel les données biométriques comparatives décrivent une empreinte anatomique du patient, dont l'image médicale est comprise par l'ensemble de données d'image médicale comparatives,
- détermination (DET-1) d'une mesure d'écart entre les données biométriques comparatives et les données biométriques,
dans lequel la mesure d'écart décrit combien les données biométriques s'écartent des données biométriques comparatives,
- affectation (CON) de l'ensemble de données d'image médicale à l'ensemble de données d'image médicale comparatives, si la mesure d'écart passe en-dessous d'une valeur de seuil.

2. Procédé suivant la revendication 1,
dans lequel l'ensemble de données d'image médicale comparatives représente au moins une partie du corps d'un premier patient et/ou dans lequel l'ensemble de données d'image médicale représente au moins une partie du corps du premier patient ou d'un deuxième patient.

3. Procédé suivant la revendication 2,
dans lequel les données biométriques comprennent au moins un repère anatomique et/ou un réseau superficiel d'au moins une anatomie de la partie du corps représentée dans l'ensemble de données d'image médicale,
dans lequel les données biométriques comparatives comprennent au moins un repère anatomique et/ou au moins un réseau superficiel d'au moins une anatomie de la partie du corps représentée dans l'ensemble de données d'image médicale comparatives.

4. Procédé suivant la revendication 3,
dans lequel le repère anatomique et/ou le réseau superficiel comprend au moins une coordonnée dans l'ensemble de données d'image médicale et respectivement dans l'ensemble de données d'image médicale comparatives.

5. Procédé suivant l'une des revendications précédentes, comprenant en outre le stade de procédé suivant :
- production (DET-2) d'une correspondance entre les données biométriques comparatives et les données biométriques,
dans lequel le stade de procédé de la détermination (DET-1) de la mesure de l'écart s'effectue sur la base de la correspondance.

6. Procédé suivant la revendication 4 et 5,
dans lequel le stade de la production (DET-2) de la correspondance comprend les stades de procédé suivants :
- détermination (DET-3) de repères anatomiques et/ou de réseaux superficiels se correspondant l'un à l'autre dans les données biométriques comparatives et les données biométriques,
- transformation (TRANS) des repères anatomiques et/ou des réseaux superficiels se correspondant l'un à l'autre dans un système de coordonnées commun,
dans lequel le stade de procédé de la détermination (DET-1) de la mesure de l'écart comprend le stade de procédé suivant :
- détermination (DET-4) d'un écart des coordonnées des repères anatomiques et des réseaux superficiels se correspondant l'un à l'autre dans le système de coordonnées commun.

7. Procédé suivant l'une des revendications 5 ou 6,
dans lequel la production (DET-2) de la correspondance entre les données biométriques comparatives et les données biométriques comprend une correspondance rigide.

8. Procédé suivant l'une des revendications précédentes,
dans lequel le stade de procédé de la détermination (DET-1) de la mesure de l'écart comprend le stade de procédé suivant :
- application (APP-1) d'une fonction entraînée aux données biométriques comparatives et aux données biométriques, grâce à quoi on détermine la mesure de l'écart.

9. Procédé suivant l'une des revendications 5 à 8 en combinaison avec la revendication 3, dans lequel une influence du au moins un repère anatomique et/ou du au moins un réseau superficiel sur la mesure de l'écart dépend de sa stabilité dans le temps.

10. Procédé suivant l'une des revendications précédentes, dans lequel le stade de procédé de l'affectation (CON) de l'ensemble de données d'image médicale comprend au moins l'un des stades suivants :
- établissement (DET-5) d'une combinaison entre l'ensemble de données d'image médicale et l'ensemble de données d'image médicale comparatives dans une base de données,
- détermination (DET-6) d'un numéro d'identification commun pour l'ensemble de données d'image médicale et l'ensemble de données d'image médicale comparatives,
- application (APP-2) d'une fonction pour l'analyse d'image à l'ensemble de données d'image médicale comparatives et à l'ensemble de données d'image médicale.

11. Procédé suivant la revendication 10,
dans lequel l'ensemble de données d'image médicale comparatives a été déterminé dans le temps, avant l'ensemble de données d'image médicale,
dans lequel, par l'application (APP-2) de la fonction pour l'analyse d'image à l'ensemble de données d'image médicale comparatives et à l'ensemble de données d'image médicale, on détermine une variation en fonction du temps entre l'ensemble de données d'image médicale comparatives et l'ensemble de données d'image médicale.

12. Procédé suivant l'une des revendications précédentes,
dans lequel l'ensemble de données d'image médicale est affecté à l'ensemble de données d'image médicale comparatives par une affectation provisoire,
dans lequel le procédé comprend en outre le stade de procédé suivant :
- correction (COR) de l'affectation provisoire entre l'ensemble de données d'image médicale et l'ensemble de données d'image médicale comparatives, si la mesure de l'écart est supérieure ou égale à la valeur de seuil.

13. Procédé suivant l'une des revendications précédentes,
dans lequel le procédé comprend en outre le stade de procédé suivant :
- lancement (PROV) d'un signal d'alerte, si la mesure de l'écart est supérieure ou égale à la valeur de seuil.

14. Système (SYS) d'affectation pour l'affectation d'un ensemble de données d'image médicale à un ensemble de données d'image médicale comparatives en fonction de données biométriques comprenant une interface (SYS.IF) et une unité (SYS.CU) informatique,
dans lequel l'interface (SYS.IF) est constituée pour la réception (REC-1) de l'ensemble de données d'image médicale,
dans lequel l'ensemble de données d'image médicale comprend une image médicale d'un patient,
dans lequel l'interface (SYS.IF) est constituée en outre pour la réception (REC-2) d'au moins un ensemble de données d'image médicale comparatives,
dans lequel l'ensemble de données d'image médicale comparatives comprend une image médicale du patient ou d'un autre patient, dans lequel l'unité (SYS.CU) informatique est constituée pour l'extraction (EXT-1) de données biométriques reposant sur l'ensemble de données d'image médicale,
dans lequel les données biométriques décrivent une empreinte anatomique du patient, dont l'image médicale est comprise par l'ensemble de données d'image médicale,
dans lequel l'unité (SYS.CU) informatique est constituée en outre pour l'extraction (EXT-2) de données biométriques comparatives reposant sur l'ensemble de données d'image médicale comparatives, dans lequel les données biométriques comparatives décrivent une empreinte anatomique du patient, dont l'image médicale est comprise par l'ensemble de données d'image médicale comparatives, dans lequel l'unité (SYS.CU) informatique est constituée en outre pour la détermination (DET-1) d'une mesure de l'écart entre les données biométriques comparatives et les données biométriques, dans lequel la mesure de l'écart décrit combien les données biométriques s'écartent des données biométriques comparatives, dans lequel l'unité (SYS.CU) informatique est constituée en outre pour l'affectation (CON) de l'ensemble de données d'image médicale à l'ensemble de données d'image médicale comparatives, si la mesure de l'écart passe en-dessous d'une valeur de seuil.

15. Programme d'ordinateur ou support de mémoire, déchiffrable par ordinateur, comportant des parties de programme pour exécuter tous les stades du procédé suivant l'une des revendications 1 à 13, lorsque les parties de programme sont exécutées par le système (SYS) .
